# EUROPEAN PATENT APPLICATION

(11) **EP 2 570 487 A1**
(43) Date of publication of application: **20.03.2013**
(21) Application number: 11181546.0
(22) Date of filing: 16.09.2011
(51) Int. Cl.: C12Q 1/68

(54) **Nucleic acid transcription method**

(71) Applicant: Lexogen GmbH, 1030 Vienna (AT)
(72) Inventor: Seitz, Alexander, 1140 Vienna (AT); Moll, Pamela, 1230 Vienna (AT); Napora, Magdalena Anna, 1160 Vienna (AT)
(74) Representative: Sonn & Partner Patentanwälte

(57) **Abstract**

The present invention relates to methods for generating an amplified nucleic acid portion of a template nucleic acid, comprising
obtaining said template nucleic acid,
annealing at least one oligonucleotide primer to said template nucleic acid,
annealing at least one oligonucleotide stopper to said template nucleic acid,
elongating the at least one oligonucleotide primer in a template specific manner until the elongating product nucleic acid reaches the position of an annealed oligonucleotide stopper,
whereby the elongation reaction is stopped, wherein in said elongation reaction said oligonucleotide stopper is not elongated, and
wherein the elongated product nucleic acid is ligated to the 3' end of said oligonucleotide stopper - said stopper may also be a primer itself - and uses and kits for performing said method.

## Description

### Field of invention

The present invention relates to the field of amplifying or analyzing samples of nucleic acids by amplification of defined sequence portions.

### Background

Numerous amplification-based methods for the amplification and detection of target nucleic acids are well known and established in the art. The polymerase chain reaction, commonly referred to as PCR, uses multiple cycles of denaturation, annealing of primer pairs to opposite strands, and primer extension to exponentially increase copy numbers of the target sequence (US 4,683,195; US 4,683,202; US 4,800,159; US 5,804,375). In a variation called RT-PCR, reverse transcriptase (RT) is used to make a complementary DNA (cDNA) from RNA, and the cDNA is then amplified by PCR to produce multiple copies of DNA (US 5,322,770; US 5, 310, 652 ) .

PCR reactions generally comprise carrying out multiple cycles of:
(A) hybridizing (annealing) a first primer to a site in a nucleic acid strand at one end of the target nucleic acid sequence, and hybridizing a second primer to a site corresponding to the opposite end of the target sequence in the complementary nucleic acid strand;
(B) synthesizing (extending) a nucleic acid sequence from each respective primer; and
(C) denaturing the double stranded nucleic acid produced in step (B) so as to form single stranded nucleic acid.
Denaturation is generally carried out at from 80 to 100°C, hybridization (annealing) is generally carried out at from 40 to 80°C, and extension is generally carried out at from 50 to 80°C. A typical cycle is denaturation: about 94°C for about 1 min, hybridization: about 58°C for about 2 min, and extension: about 72°C for about 1 min. The exact protocol depends on factors such as the length and sequence of the primers and target sequence, and the enzyme used.

PCR has been adopted for various applications. E.g., the GB 2293238 describes methods to reduce non-specific priming and amplifying nucleic acid sequences. Blocking "primers" (or oligonucleotides) are disclosed that produce misalignment and reduce non-specific priming by creating competitive primer annealing reactions to the amplification primers. For example, mixtures of random blocking primers are used that comprise a ddNTP at the 3' position to prevent initiation of extension reactions. Only correct amplification primers displace their blocking primers and can initiate the amplification reaction.

Methods have been established for using blocking primers that specifically bind to unwanted target oligonucleotide molecules in a sample to prevent amplification thereof in a PCR reaction of unblocked oligonucleotide molecules. Unblocked oligonucleotides can be amplified without further measures to ensure target specificity - in the absence of amplifiable competitive oligonucleotide molecules that are not intended for amplification (US 2002/0076767 A1 and US 6,391,592 B1; WO 99/61661).

A similar method is disclosed in the WO 02/086155, wherein blocking oligonucleotides bound to an undesired template result in premature termination of an elongation reaction. The blocking oligonucleotides bind specifically to one template in a mixture while leaving other templates free for amplification.

US 5,849,497 describes the use of blocking oligonucleotides during a PCR method with a DNA polymerase lacking 5' exonuclease activity. This DNA polymerase cannot digest the blocking oligonucleotides that prevent amplification. Such a system has been selected to avoid using PNA (peptide nucleic acids) as blocking oligonucleotides. A similar system is described in WO 2009/019008 that however contemplates the use of PNA and LNA, among others, as blocking oligonucleotides.

All these methods have in common that amplification of unwanted templates is specifically suppressed by hybridization of a specific blocking oligonucleotide.

The analysis of RNA regularly starts with reverse transcribing RNA into cDNA as DNA is more stable than RNA and many methods exist for analyzing DNA. Whatever protocol is used to analyze the cDNA, it is important that the cDNA generated during reverse transcription (RT) represents the RNA that needs to be analyzed in sequence and concentration as closely as possible.

Reverse transcription is generally carried out using reverse transcriptases. These enzymes require an oligonucleotide primer that hybridizes to the RNA to start (prime) the template dependent polymerization of the cDNA. The two most common priming strategies used are oligo dT priming and random priming.

Oligo dT priming is used for RT of mRNAs that have a poly A tail on their 3' end. The oligo dT primes the RNA at the 3' end and the reverse transcriptase copies the mRNA up to its 5' end. One drawback of this approach is that high quality mRNA is needed as any mRNA degradation will lead to a strong overrepresentation of the 3' ends of mRNAs.

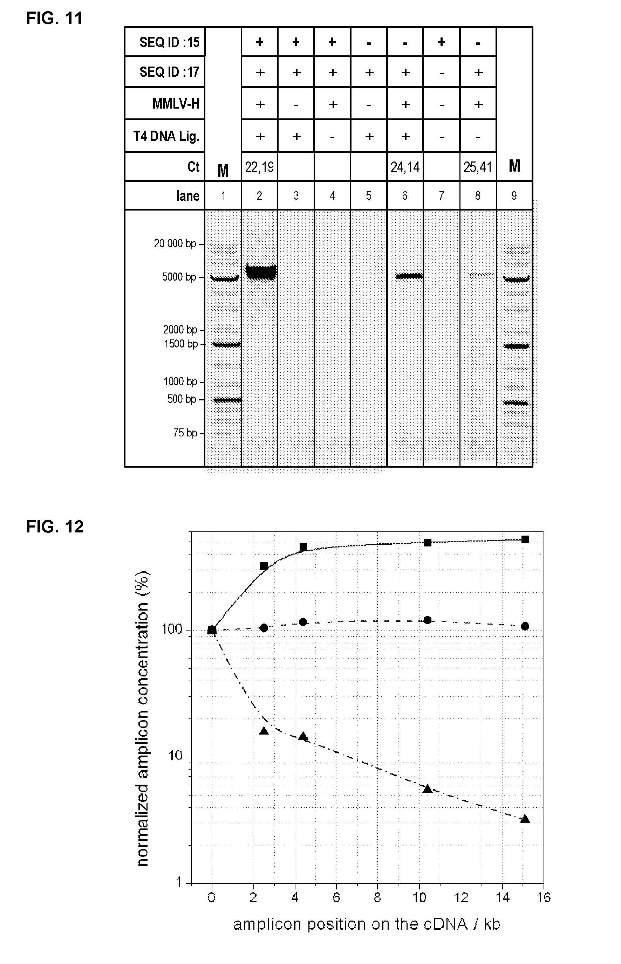

Even if un-degraded mRNA is used the cDNA molecules may still be truncated due to premature polymerization stop events. A frequent cause is secondary and tertiary structure formation in highly structured RNA regions. Especially when the GC content is high the reverse transcriptase might not read through these regions and thus the cDNA becomes truncated. The likelihood of such events to occur increases the longer the mRNA is that needs to be copied. Therefore oligo dT primed cDNA can show a strong bias towards over-representing the 3' ends of RNAs. Thus, 3' end priming suffers from a concentration bias that leads to an increase of sequences at or near the 3' end with gradually reduced representation of sequences in the direction of the 5' end (see Fig. 12, triangles, for qPCR measurement of the bias). This is problematic in quantitative approaches, e.g. in the determination of the degree of expression of a particular gene, in difference analysis or in complete expression profiling of a cell.

Approaches have been developed to overcome RNA secondary structure termination especially when long mRNAs need to be reverse transcribed into full length cDNAs. One such method for instance involves a mixture of 2 reverse transcriptases, one highly processive such as MMLV or AMV and mutants thereof, first incubating the reaction mixture at a normal temperature range to allow first strand synthesis plus using a thermostable enzyme composition having reverse transcriptase activity and then incubating the reaction mixture at a temperature that inhibits the presence of secondary mRNA structures to generate a first strand (United States Patent US 6,406,891). However, buffers for reverse transcriptases contain high concentrations of MgCl₂ (3-10 mM) or Mn²⁺ (e.g for Tth DNA polymerase) and RNA is highly unstable and susceptible to breaks and/or degradation at higher temperatures especially in the presence of these divalent cations. The cycling method between two temperatures to bypass secondary structures might also lead to random priming by short RNA fragments that were generated during the high temperatures. Such short RNA fragments will be used by the MMLV-H or other viral reverse transcriptases as a primer [1]. Again this would lead to a bias in the synthesized cDNA.

Another approach is random priming that has the advantage of hybridizing at multiple locations along the RNA and hence also blocking those sequences from taking part in secondary structure formation. In random priming an oligonucleotide population of random sequence, usually a random hexamer, is used to prime the RT anywhere within the template nucleic acid strand. Random priming is used for both reverse transcription or regular transcription using DNA as template. When product DNA was analyzed it was found that random priming does not result in equal efficiencies of reverse transcription for all targets in the sample [2, 3]. Furthermore there is no linear correlation between the amount of template nucleic acid input and product DNA output when specific targets are measured [2, 3]. Indeed, it has been shown that the use of random primers can lead to overestimate some template copy numbers by up to 19-fold compared to sequence-specific primed templates [4]. Although a lot was speculated about the underlying causes for these phenomena, no conclusive rational has been put forward.

### Objective

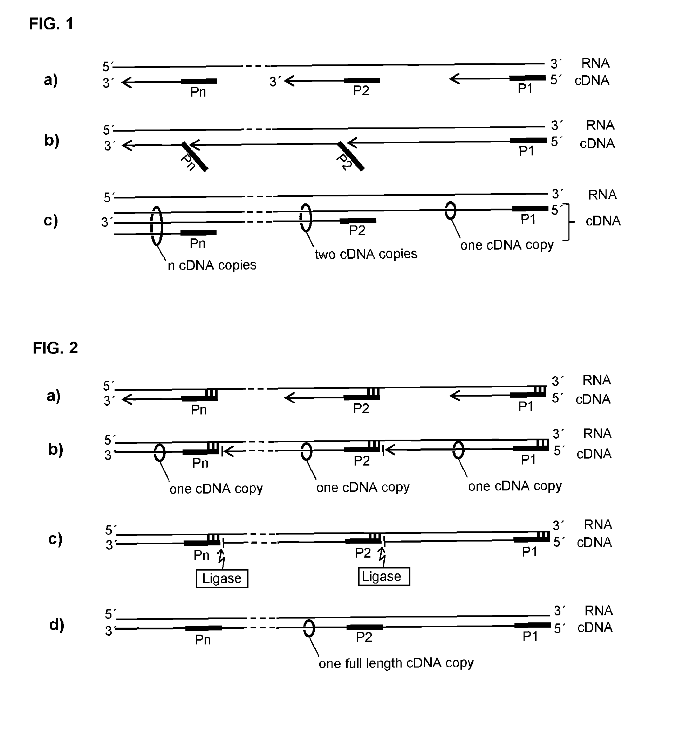

The present inventors have observed that there is a general bias in such randomly primed cDNA libraries, in the form that the sequence parts on the 5' ends of RNA molecules are overrepresented when compared to the parts on 3' ends. The reason for this phenomenon is to be found in the combination of random priming and the strong strand displacement activity of the reverse transcriptases. As RNA has a high degree of secondary structure, reverse transcriptases had to evolve a strong strand displacement activity to overcome this secondary structure and to effectively generate cDNA. Given the strong strand displacement activity of reverse transcriptase the 5'side of any RNA will be represented several times in a cDNA library when random oligonucleotides are used for priming. A similar effect happens during extension of (e.g. random) primer combinations having primers that anneal to a more 3' position on the template RNA (upstream primers in the direction of the extension products), wherein the reverse transcriptase will displace the extension products of all primers that have hybridized to a more 5' position on the template RNA (downstream primers in the direction of the extension products). Therefore random priming is a DNA synthesis method with a strong bias to overrepresent the 5' ends of a given template nucleic acid (see also Fig. 1 for a schematic representation of the problem and Fig. 12 for qPCR measurements of the bias). Besides using randomers (e.g.: random hexamers) for cDNA library preparation and in radio labeling of DNA probes [5, 6], they are also used to detect Single Nucleotide Polymorphisms (SNPs) as well as small scale chromosome events, primarily insertions or deletions [3, 4]. Comparative Genomic Hybridization (CGH) has been developed to elucidate genome-wide sequence copy-number variation (CNV) between different genomes, such as the differential amplification or deletion of genetic regions between tumor DNA and normal DNA from neighboring unaffected tissue [7, 8] .

Currently one of the most complete analysis methods for DNA libraries is Next Generation Sequencing (NGS) [for review see 9] . NGS is a generic term for parallel sequencing through polymerization in a high throughput manner. NGS is based on obtaining sequencing reads from small fragments. In the generation of cDNA libraries, either the mRNA is fragmented before cDNA synthesis or single stranded or double stranded cDNA is fragmented. However, any fragmentation of template nucleic acids (chemical or physical) introduces an undefined and not foreseeable bias and will deplete the template. In NGS, the complete sequence is obtained by alignment of those reads which is a challenging task due to the sheer number of small reads that have to be assembled to a complete sequence. To date many reads provide just limited information. For instance many of the reads cannot be assigned uniquely and therefore are discarded. Sequence generation is further hindered by representation bias of sequence fragments.

Therefore there is the need for improved methods for amplifying template nucleic acids that yield less bias in the amplified amount, e.g. for NGS or for the generation of DNA libraries to improve representation of the sequence concentration of the original template.

### Summary of the invention

Therefore the present invention provides a method for generating an amplified nucleic acid portion of a template nucleic acid, comprising
obtaining said template nucleic acid,
annealing at least one oligonucleotide primer to said template nucleic acid,
annealing at least one oligonucleotide stopper to said template nucleic acid,
elongating the at least one oligonucleotide primer in a template specific manner until the elongating product nucleic acid reaches the position of an annealed oligonucleotide stopper, whereby the elongation reaction is stopped, wherein in said elongation reaction said oligonucleotide stopper is not elongated, and
wherein the elongated product nucleic acid is labelled at the 3' end at a position adjacent to said oligonucleotide stopper and/or wherein the elongated product nucleic acid is ligated to the 5' end of said oligonucleotide stopper; thus obtaining an amplified nucleic acid portion.

In a further aspect the present invention provides a method of generating an amplified nucleic acid of a template nucleic acid, comprising
obtaining said template nucleic acid,
annealing a first oligonucleotide primer to said template nucleic acid,
annealing at least one further oligonucleotide primer to said template nucleic acid,
elongating said first oligonucleotide primer in a template specific manner until the elongating product nucleic acid reaches the position of one of said further oligonucleotide primers, whereby the elongation reaction is stopped, and at least one further oligonucleotide primer is elongated in a template specific manner, wherein the elongated product nucleic acid is labelled at the 3' end at a position adjacent to said further oligonucleotide primer and/or wherein the stopped elongated product nucleic acid is ligated to the 5' end of said further oligonucleotide primer; thus obtaining an amplified nucleic acid portion. In this method said further oligonucleotide primer serves both as a stopper, which prevents further elongation of an amplification reaction that reaches the position of the annealed stopper, and as a primer itself, i.e. as an initiator of elongation.

The template nucleic acid can comprise or substantially consist of RNA or DNA, in preferred embodiments said template is RNA.

In a preferred aspect the present invention provides a method of generating an amplified nucleic acid of a template RNA, comprising
obtaining said template RNA,
annealing a first oligonucleotide primer to said template nucleic acid,
annealing at least one further oligonucleotide primer to said template RNA,
elongating said first oligonucleotide primer in a template specific manner until the elongating product nucleic acid reaches the position of one of said further oligonucleotide primers, whereby the elongation reaction is stopped, and at least one further oligonucleotide primer is elongated in a template specific manner.

In a further aspect, the present invention provides the use of the inventive methods to generate a sequence library of one or more template nucleic acids comprising a mixture of, preferably overlapping, amplified nucleic acid portions of said template nucleic acids. A sequence library is a collection of DNA fragments that can be stored and copied through any process known in the art. For instance a sequence library can be obtained through the process of molecular cloning. Sequence libraries can also be amplified through e.g. PCR using universal sequences on the ends of the DNA fragments.

The invention also relates to a kit for generating amplified nucleic acid portions of a template nucleic acid or for generating a sequence library as mentioned above. An inventive kit comprises a reverse transcriptase, random oligonucleotide primers which comprise a modification that increases the Tm (melting temperature) and random oligonucleotide stoppers that are unsuitable for nucleotide extension and comprise a modification that increases the Tm, optionally further one or more of reaction buffers comprising Mn²⁺ or Mg²⁺, a DNA ligase, PEG.

The following detailed disclosure reads on all aspects and embodiments of the present invention.

### Detailed disclosure of the invention

The present invention provides a method for generating an amplified nucleic acid or amplifying nucleic acids. This generation can relate also to a single amplification reaction, e.g. one transcription cycle, or more. It includes the generation of RNA or DNA by RNA or DNA dependent polymerization. Thus, amplifying nucleic acids included polymerization of RNA nucleotides based on an RNA or a DNA template nucleic acid or the polymerization of DNA nucleotides based on an RNA or a DNA template nucleic acid. Preferably the method includes one step or cycle of reverse transcription, RNA dependent DNA polymerization.

The inventive methods include the use of at least two short oligonucleotides that hybridize with the template nucleic acid. At least one oligonucleotide has a primer function, i.e. it can act as nucleotide polymerization initiator for polymerase dependent amplification, i.e. transcription. The extension of primers by the addition of nucleotides in a template dependent fashion is referred herein as elongation or extension. The products of such reactions are called elongation products or extension products. RNA or DNA polymerases add nucleotides to given oligonucleotide strand which base pair to a nucleobase of a template strand. Hybridization and annealing is understood as base pairing of complementary nucleotides. Complementary nucleotides or bases are those capable of base pairing such as A and T (or U); G and C; G and U.

At least one further oligonucleotide has a stopper function. This means that as an elongation (extension) reaction that has been initiated at an upstream primer (in the direction of the extension products) reaches a downstream (in the direction of the extension products) oligonucleotide with a stopper function, said elongation reaction is prevented from further elongation. Relative to the nucleotide position on the template nucleic acid this means that once an elongation reaction that has been initiated from a primer that has annealed more to the 3' end of the template nucleic acid relative to the oligonucleotide with the stopper function, reaches that oligonucleotide (stopper), said elongation reaction is prevented from further elongation. The elongation reaction can be stopped by strong hybridization of the oligonucleotide with stopper function to the template nucleic acids so that it is not displaced by the polymerase.

The oligonucleotide with stopper function can also be a primer. It can hybridize downstream (in the direction of the elongation reaction, upstream in the direction of the template) to a first primer and stop the elongation reaction of said first primer. In turn (or simultaneously) it acts as elongation initiator itself to produce a transcription product - that in turn may also be stopped at the position of a further downstream (in relation to the direction of the elongation reactions) oligonucleotide with stopper function.

"Upstream" relates to the direction towards the 5' end (3'-5') of a given nucleic acid or oligonucleotide. "Downstream" relates to the direction towards the 3' end (5' -3') of a given nucleic acid or oligonucleotide. Since oligonucleotides hybridize in inverse fashion downstream for a primer relates to the upstream direction of a hybridized template nucleic acid. This means that a downstream oligonucleotide (or oligo, or primer or stopper or blocker) is an oligo that hybridized to a more upstream portion of a template nucleic acid in relation to an upstream oligonucleotide (or oligo, or primer or stopper or blocker) that has hybridized to a more downstream portion of a template nucleic acid. Therefore, when using the term "downstream oligonucleotide", or "upstream oligonucleotide" the directionality always refers to that of the extension product(s), except when stated otherwise. The polymerase dependent elongation reactions of the invention are in 5'-3' direction, downstream.

As used herein, "comprising" shall be understood as referring to an open definition, allowing further members of similar or other features. "Consisting of" shall be understood as a closed definition relating to a limited range of features.

As used herein "primer" may also refer to an oligonucleotide primer. "Stopper" refers also to oligonucleotide stopper. "Oligo" is used for both oligonucleotide primers and oligonucleotide stoppers.

An oligonucleotide stopper is an oligonucleotide that can stop an elongation reaction as described above and does not initiate a further elongation reaction, e.g. the oligonucleotide is incapable of accepting (covalently binding) a further nucleotide at its 3' position. Such nucleotides are known in the art and usually lack a 3' OH, as e.g. in ddNTS (dideoxynucleotides).

Thus, the present invention essentially provides two methods, one utilizing oligonucleotide stoppers and one further oligonucleotide primers. Apart from this difference, the methods comprise the inventive similarity to provide limited and well defined amplification products that can be amplified in well controlled fashion and most importantly, with a unitary concentration distribution over the length of the template nucleic acid. The inventive amplification products can be further characterized and used. They are the desired products that are obtained and not discarded, like in template suppression methods. The present invention generates amplified nucleic acid products that better represent the template molecules that need to be analyzed and prepares those for seamless integration with subsequent analysis methods such as next generation sequencing or as sequence library. If the stoppers are at the same time primers, it is possible to provide one continuous sequence based on only one template molecule. This continuous sequence of the amplified product can be provided as single molecule when the individual product sequences are covalently connected, e.g. ligated. Such connection or ligation reaction can be performed while hybridized to the template strand to secure that the products are connected in the same order as the template strand (while of course being the reverse complement strand).

The advantages of the present invention are most prominent with long template nucleic acids that are amplified according to the invention. Such templates may be at least 100 bases, at least 1000 bases (1kb), at least 2 kb, at least 4 kb, at least 6 kb, at least 10 kb, at least 20 kb, at least 30 kb, at least 40 kb, at least 50 kb, in length.

In case of a reverse transcription an embodiment of the present invention relates to a method of reverse transcribing an RNA molecule comprising hybridizing at least two primers to a template RNA molecule and extending primers utilizing an RNA dependent DNA polymerase, wherein the extension product of an upstream primer (downstream in the direction of the template) does not displace the extension product of a downstream primer (upstream in the direction of the template). This embodiment in general essentially comprises a primer extension reaction using a first primer that is extended but stops at (does not displace) a second primer that is also extended.

However, for a complete representation of a given template by the elongation product, direct connection of the elongated nucleotides is not necessarily required. A sample of template nucleic acids usually contains many template molecules that have the same sequence. By using many, more than one, primer and stopper combinations it is possible to have a complete representation of such a sequence by the many elongation products. Having many short elongation products is often a requirement of a nucleic acid library that fully represents the template. Thus the present invention provides the method of preparing a nucleic acid library by providing the elongation products. The library may contain the elongation products in a mixture. Preferably the elongation products, especially of templates of the same sequence, contain overlapping sequence portions. Overlapping sequence portions are easier for complete sequence assembly, as e.g. required in NGS methods and are desired to provide a library that can be used to clone any specific sequence therein without or with limited interruptions by reaching the size limits of an individual elongated nucleic acid.

In case of a reverse transcription such an embodiment of the present invention relates to a method of reverse transcribing an RNA molecule comprising hybridizing at least two oligonucleotides, one a primer the other a stopper, to a template RNA molecule and extending the primer utilizing an RNA dependent DNA polymerase, wherein the extension product of the upstream primer (downstream in the direction of the template) stops at (does not displace) the position of the downstream stopper (upstream in the direction of the template). Said stopper is not extended. This embodiment in general essentially comprises a primer extension reaction using a first primer that is extended but stops at (does not displace) an oligonucleotide stopper that is not extended.

It is possible to combine the inventive embodiments, e.g. by using at least two primers and a stopper, a first upstream primer that is extended downstream, which extension reaction stops at the position of a second, downstream primer that is also extended downstream, which extension reaction in turn stops at the position of the oligonucleotide stopper.

In special embodiments no oligonucleotide stoppers that cannot be extended are used. In such embodiments only extendable primers may be used during amplification/transcription.

In other embodiments it is desired to obtain amplification products that have been stopped at oligonucleotide stoppers. To this end a skilled man can e.g. select such elongated nucleic acids by well-known methods, such as by labeling, including immobilization onto a solid phase (e.g. beads or a solid surface) or by attaching a barcode sequence or sequence tag. The elongated products may also be ligated to the oligonucleotide stopper - similar to the ligation with primers with stopper function in order to provide one long product molecule as mentioned above. Labeling and ligating to the oligonucleotide stopper can be combined, e.g. by labeling the oligonucleotide stopper and ligating the extension product to a labeled oligonucleotide stopper. Of course, labeling of the oligonucleotide stopper can be performed after ligation with the extension product. Such labeling allows the easy handling, selection and/or amplification of the elongated products. E.g. a sequence tag can be used for further selected amplification of said elongated and so labeled elongation products by using primers that hybridize to such a tag and can initiate an amplification reaction, e.g. PCR, of said (previously) elongated nucleic acid product. This is particularly advantageous if a multitude of different elongation products are obtained and many are labeled with the same sequence tag. In case of sequence libraries this method allows an easy amplification of an entire library - still with a consistent representation of the amount of all sequences over the entire length of the original template.

In preferred embodiments more than one oligonucleotide primer is used, which functions similar as the first primer but has a different primer sequence, the sequence that anneals to a target template. In particular the inventive method according to this embodiment further comprises annealing an additional oligonucleotide primer to said template nucleic acid and elongating said additional oligonucleotide primer until the elongating product nucleic acid reaches the position of another oligonucleotide primer or an oligonucleotide stopper. In especially preferred embodiments at least 1, at least 2, at least 3, at least 4, at least 5, at least 6, at least 7, at least 8, at least 9, at least 10, at least 15, at least 20, at least 30, at least 40, at least 50 or more different oligonucleotide primers are used. "Different oligonucleotide primers" is understood that they differ in a primer sequence but, of course, may share other similar sequence portions, such as sequence tags. Such sequence tags are preferably used in a further amplification reaction of the elongated products by using primers that anneal to the sequence tags. Thus, with one primer all potentially different products can be amplified. In a special embodiment the oligonucleotide primers are random primers. "Random primers" is to be understood as a mixture of different primers with different primer sequence portions, with a high variance due to a random synthesis of at least a portion of the primer sequence. Random primers potentially cover the entire combinatory area for said sequence. The random sequence primer portion of the random primer may cover 1, 2, 3, 4, 5, 6, 7, 8 or more nucleotides which are randomly selected from A, G, C or T (U). In terms of hybridizing sequences of primer sequences T and U are used interchangeably herein. The full combinatory possible area for a random sequence portion is mⁿ, wherein m is the number of nucleotide types used (preferably all four of A, G, C, T(U) and n is the number of the random nucleotides. Therefore a random hexamer, wherein each possible sequence is represented, consists of 4⁶ = 4096 different sequences.

Likewise it is also possible to use more than one oligonucleotide stopper in any one of the inventive methods. Said additional stoppers act similar as the first stopper, but differ in the sequence aligned to the template. The same as described above for additional primers applies for the additional stopper - of course with the difference that the stoppers are not suitable for elongation reactions. Therefore, for consistency the region of the oligonucleotide stopper that hybridizes to the template is also referred to as "primer sequence". Said primer sequence may be 4, 5, 6, 7, 8, 9, 10, 11, 12, 13, 14, 15, 16, 17, 18, 19, 20, 21, 22 or more nucleotides long. The invention thus provides a method as defined above further comprising annealing an additional oligonucleotide stopper to said template nucleic acid and wherein in said elongation reaction said additional oligonucleotide stopper is not elongated. In preferred embodiments at least 1, at least 2, at least 3, at least 4, at least 5, at least 6, at least 7, at least 8, at least 9, at least 10, at least 15, at least 20, at least 30, at least 40, at least 50 or more different oligonucleotide stoppers are used. The oligonucleotide stoppers may be random oligonucleotide stoppers, comprising a random primer sequence that anneals to the template. As described above for oligonucleotide primers, also oligonucleotide stoppers may share similar sequence portions, such as tags or barcodes, that may be used for amplification or identification of the products. Such kinds of labels allow easy identification in a sequence library comprising the inventive amplification products.

As described in the introduction a (randomly) primed cDNA library without control of the elongation reaction (and its stop) distorts the actual RNA representation of sequence portions. Even on e.g. short mRNA templates (200-1000nt) multiple priming events can occur and with the strand displacement the 5' side of RNA molecules will be present in more copies than the 3' side (see also Fig. 1). Therefore when measuring the concentration of a certain gene transcript different values will be obtained when probing for sequence portions at the 5' or 3' end. Short random probes are commonly used in microarray and quantitative PCR analysis. This leads to severe distortions of the concentration measured. Furthermore, this distortion will be greater when comparing long and short transcripts, as the 5' ends of high abundant long transcripts will be even higher represented than the 5' ends of shorter transcripts. When globally analyzing the concentration of transcripts such as in high throughput sequencing (e.g. next generation sequencing) in addition to distorting concentrations detecting rare short transcripts becomes even less likely than detecting rare long transcripts. As the differential expression of gene transcripts and their splice variants is an important part of phenotype analysis, it is important that each sequence portion of a transcript is represented in the generated cDNA library at the correct abundance.

These problems are solved by the present invention. Applying the inventive methods to provide well defined transcripts, stopped at a primer or stopper position (preferably with inhibited strand displacement of the reverse transcriptase) during the generation of cDNA from a multitude of RNA molecules as for instance in the generation of a cDNA library of mRNA molecules, enables the equal representation of each portion of the RNA molecules. For instance mRNA can be primed with random primers such as random hexamers that are modified to ensure that the random primer does not get displaced by the reverse transcriptase. Any random primer can be used that can start reverse transcription from multiple sites from the template RNA molecules.

The present invention also provides for methods that enable the covalent joining (e.g.: through a ligation) of the obtained elongated products as they are provided in a hybrid with the template (see also Fig. 2c). This enables the generation of a full length amplified nucleic acid molecule.

Using primers that provide for a 5' phosphate, it has been found that the "short" elongated nucleic acids - that are essentially fragments of the complementary strand to the template strand - can be ligated while still being hybridized to the template. This will result in long and in most cases full length amplified nucleic acid molecules that are a direct representation of the template. Being able to preserve the information of the full length sequence of a template is, for instance, important when splice variants of a gene need to be analyzed. Especially when splicing of multi exonic genes is complex the analysis of single splice junctions alone will not yield unambiguous information towards the full length sequence of the transcript variant involved. However, only oligo dT priming in case of mRNA or priming from a 3' ligated universal linker from any nucleic acid template will lead to an underrepresentation of the 5' side of the template. In other words, the longer the template is the less likely it becomes that the molecule will be reverse transcribed to full length. As can be seen in Fig. 12, when a standard oligo dT primed RT is used on average after 6kb only ∼10% of RNA molecules are reverse transcribed to full length. As the present invention provides - in one embodiment - for methods that will start the polymerization from two or more positions of a template molecule with an elongation reaction that is stopped at the position of a downstream primer, the extension product of the upstream primer can be ligated to the 5' end of the downstream primer. By covalently joining both extension products when in hybrid with the template a longer sequence is created. In continuation when many different primers are used potentially all templates present in the sample can be transcribed and by ligating the short extension products a full length amplified copy of the template can be created (see also Fig. 2c, d).

Likewise, according to the inventive embodiments utilizing oligonucleotide stoppers, a ligation with the elongated product can be performed to obtain well defined amplified nucleic acids of an amplification of a sequence between the primer and the stopper.

In preferred embodiments the oligonucleotide primers and/or oligonucleotide stoppers are phosphorylated or adenylated on the 5' end. This measure helps to easily ligate the oligonucleotide primers or stoppers to another nucleic acid, such as the elongation product, in one or few steps using a ligase. In some embodiments, especially when mixtures of many primers and stoppers are used, it is possible to only provide the stoppers with such a modification to ensure ligation of elongated nucleic acids with stoppers and prevent ligation with other primers.

To prevent any non-hybridized primers or stoppers from being ligated a preferred embodiment of the invention uses T4 DNA ligase. Double stranded DNA is the natural substrate of T4 DNA ligase and DNA-RNA hybrids are poor substrates [27]. In order to overcome this inefficiency Mg²⁺ can be replaced with Mn²⁺ as a divalent ion for the enzyme [28]. In one embodiment of this invention the addition of PEG to the ligation reaction is shown to increase the ligation efficiency of DNA molecules in an RNA hybrid even in a Mg²⁺ containing ligase buffer.

Optionally T4 RNA ligase, which is deficient of adenylation (e.g. truncated), can be used for ligation which relies on the presence of adenylated DNA fragments that are in the hybrid with the RNA (adenylation with T4 DNA ligase occurs exclusively in double stranded nucleic acids). It can be added additionally to the ligation reaction to further increase the efficiency. The ligation happens exclusively in the hybrid and was previously considered very inefficient form of ligation [29]. In US 6,368,801 a ligation of 2 DNA molecules (with ribonucleotides at their 3' and 5' ends) in an RNA hybrid by T4 RNA ligase is described. However T4 RNA ligase is not specific for hybrids since it ligates all single stranded nucleic acids containing deoxyribonucleotides at their 5' or 3' end (RNA to DNA, RNA to RNA, DNA to DNA providing there is one deoxyribonucleotide at the 3' and 5' end) as well. The present invention includes the addition of PEG in the ligation reaction, which allows the ligation to take place in the RNA hybrid. PEG has been used in single stranded ligation reactions as a molecular crowding agent, increasing the likelihood of the donor oligonucleotide ligase complex interacting with the acceptor (3'OH) by decreasing the effective reactive volume [30]. Here in contrast the donor oligonucleotide ligase complex is already next to the acceptor and PEG serves to change the conformation of the RNA:DNA hybrid by condensing the double helix into a conformation that is more reminiscent of a DNA:DNA helix, so that the T4 DNA ligase that normally is specific for ligating two DNA molecules in a hybrid with a DNA strand, can ligate two DNA molecules that are in a hybrid with an RNA molecule.

Other additives such as Tween-20, NP-40 could be added additionally or instead of PEG for efficient ligation in the RNA hybrid. Within the scope of the invention the ligation reaction requires 12%-25% final PEG-8000 (v/v). A variety of PEG molecular weights and compounds can be used, and the skilled experimenter will appreciate that the identity and concentration of the additive can be varied to optimize results. In the context of the present invention, an effective amount of PEG is an amount sufficient to permit ligation activity in an RNA hybrid. In a 20 µl RT reaction the optimal PEG concentration for a ligation in an RNA hybrid was found to be 20%. However, it is apparent to the skilled in the art that optionally the reaction volume and the PEG concentration can be increased to potentially further optimize the ligation efficiency of 2 DNA molecules in an RNA hybrid by T4 DNA ligase. Other additives such as 1mM HCC and/or pyrophosphatase can further increase the ligation efficiency in an RNA hybrid.

In the presence of Mn²⁺ adenylation of primers and stoppers in a hybrid is also happening without any ligase being added although at a much slower rate. This feature can optionally be used to achieve a ligation of DNA primers or stoppers in an RNA hybrid with truncated T4 RNA ligase 2. Since this ligase is deficient of performing an adenylation by itself it won't be able to ligate any nucleic acids that are not in a hybrid. Alternatively pre-adenylated oligonucleotides can be inserted into the reaction and used with truncated T4 RNA ligase, provided that any unhybridized oligonucleotides have been removed prior to the ligation reaction. As mentioned before in another embodiment of this invention truncated T4 RNA ligase 2 can be added in addition to T4 DNA ligase to further boost the ligation of DNA fragments in an RNA hybrid.

Therefore it is preferred that the ligase is a T4 DNA ligase and optionally T4 RNA ligase 2 is added and wherein it is preferred that Polyethylene glycol is used at a concentration between 12% and 25%.

Apart from a representative template amplificate synthesis an additional benefit of the present invention is the improved efficiency of generating long product nucleic acids, especially cDNA synthesis, resulting in higher sensitivity of detection and longer products (see Example 5 and 6).

In preferred embodiments any one of the oligonucleotide primers may comprise a sequence tag. Such a sequence tag is a label in form of a unique pre-selected nucleic acid sequence that can be used to detect, recognize or amplify a sequence labelled with said tag. Preferably a uniform sequence tag is attached to more than one of the oligonucleotide primers, especially preferred to all of the oligonucleotide primers. Such a sequence tag is preferably prevented from annealing to the template, e.g. by being hybridized to a complementary nucleic acid. Sequence tags can be attached to the 5' end of the oligonucleotide stopper - so as not to prevent the elongation reaction of the primer at its 3' end.

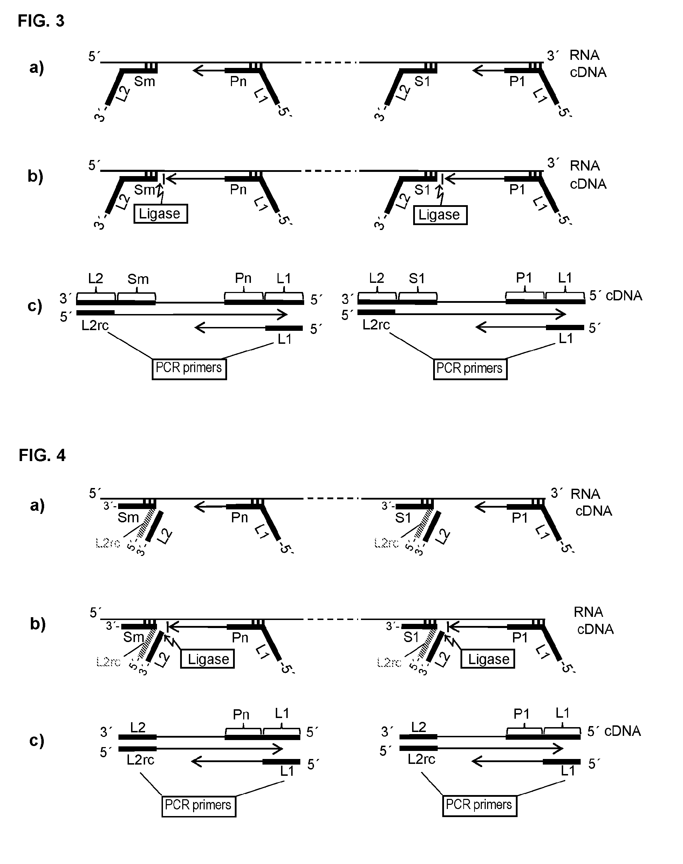

In preferred embodiments any one of the oligonucleotide stoppers may comprise a sequence tag. Such a sequence tag is a label in form of a unique pre-selected nucleic acid sequence that can be used to detect, recognize or amplify a sequence labelled with said tag. Preferably a uniform sequence tag is attached to more than one of the oligonucleotide stoppers, especially preferred to all of the oligonucleotide stoppers. Such a sequence tag is preferably prevented from annealing to the template, e.g. by being hybridized to a complementary nucleic acid. Sequence tag can be attached to the 3' end of the oligonucleotide stopper. At this position the tag does not hinder the contact of the elongating product nucleic acid to the 5' end of the stopper - resulting in well-defined products. It also allows ligation of the elongated product to the oligonucleotide stopper. Alternatively the tag may be on the 5' end of said stopper, however, prevented from hybridization to the template so that the elongation reaction still reaches the 5' end of the primer region of the stopper. Said tag preferably comprises a free 5' end so that the elongated product can be ligated to the tag for labelling of the elongated product by said tag. A free 5' end of the tag that can be easily ligated to the product is preferably provided in the vicinity of the 3' end of the elongated product. This can be achieved by e.g. providing the tag hybridized to a complementary region of the oligonucleotide stopper, said complementary region being hybridized with the tag and attached to the 5' end of the primer region of the oligonucleotide stopper (for an example see Fig. 4).

The inventive labelling step of the elongated product when it reaches the position of the oligonucleotide stopper (or another primer), preferably a stopper, can be achieved with any known means readily available in the art. Such means include attaching a chromophore, fluorophore or simple phase separation by binding to a solid phase and washing of said solid phase to remove all non-bound nucleic acids, thereby isolating the labelled nucleic acid. In preferred embodiments said labeling step comprises ligation with a sequence tag. A sequence tag may be attached to said oligonucleotide primers or oligonucleotide stoppers. Labelling may also comprises ligation with said oligonucleotide primers or oligonucleotide stoppers, which comprise a sequence tag.

For many downstream analyses it is preferred that oligonucleotides, such as primers, stoppers, blockers or linkers get depleted. Especially, when an amplification of the library utilizing the universal linker sequences is necessary or desired it is preferable to deplete the un-ligated linkers.

This can be achieved through for instance a size exclusion, retaining the oligonucleotides (shorter) in an appropriate bed, and recovering the library (longer). Another possibility is to bind the longer library to a silica based carrier while not retaining the shorter oligos. Such discrimination can also be carried out to distinguish between the single strandedness of the oligo and the double strandedness of the library when still in hybrid with the template. Another possibility for length based purification are methods based on PEG precipitation. The higher the PEG concentration the shorter the nucleic acids that can be precipitated. For instance, it was found that using a 12.5% PEG precipitation, all small fragments (below 60nts) will stay in the solution and only the cDNA and the linker ligated library will precipitate.

In a preferred embodiment a beads-based clean-up approach is used. Oligo dT coupled beads or Streptavidin beads to isolate biotin labeled oligodT primers are commercially available. A beads based clean up has the additional advantage that both RT and ligation can be performed on the beads. After hybridizing the mRNA to the oligo dT (biotin tagged or on beads) and to the starter and stoppers, any non-hybridized starter and stopper excess can be washed off before starting the RT reaction. Thus in preferred embodiments of the invention the template nucleic acid is immobilized on a solid phase or solid support, preferably beads. The amplified nucleic acids hybridized to said template nucleic acid may then be washed.

Another embodiment of the invention is that RT and ligation can be performed in one reaction step, simply by adding T4 DNA ligase, 10% PEG, and 0.4 µM ATP to the reverse transcription reaction in a regular reverse transcription reaction buffer. Although 30min incubation can be used, a better yield was obtained using 2h incubation at 37°C. Following another washing step (4 washes) the RNA can then be hydrolyzed to obtain the di-tagged cDNA library, which can then be inserted into a PCR reaction. RT and ligation can also be performed in two successive reaction steps, re-buffering the reaction simply by washing the beads. However, in a preferred embodiment of the invention a simultaneous RT/ligation reaction is performed since this resulted in similar yield as the two step protocol, but has the advantage of reduced hands on and incubation times. Simultaneous RT and ligation can be performed by addition of a DNA polymerase and DNA ligase in one reaction mixture with the template nucleic acid.

In preferred methods of the invention said elongated products are amplified. Amplification may comprise using tag specific primers to amplify elongated products that comprise a 5' and/or 3' tag, stemming from the tag-labelled oligonucleotide primer and/or stopper, respectively. Amplification is preferably by PCR. Sequence tags suitable for primer hybridization in a following amplification cycle are also referred to herein as "linkers".

Currently any preparation of small cDNA fragments for high throughput sequencing such as NGS involves fragmentation of RNA and in most cases a multistep procedure to introduce the 5' and 3' linker tags for amplification and bar-coding. For instance Epicentre's ScriptSeq^{™} mRNA-Seq Library Preparation Kit uses terminal-tagging technology (US 2009/0227009 A1 ) and randompriming on chemically fragmented RNA (depleted of ribosomal RNA). However, any fragmentation of mRNA (chemical or physical) introduces an undefined and not foreseeable bias. In addition during fragmentation protocols a portion of the RNA is degraded or gets lost. Therefore the present invention is ideally suited to generate cDNA libraries of a rather defined short size without the need of RNA fragmentation. Of course this method can be employed for the generation of fragments to any kind of template nucleic acid and is not limited to RNA. Such fragments are provided by the inventive elongated products or amplified nucleic acid portions. The template nucleotide sequence could also be DNA. Hence a library preparation using the techniques described in this invention can also be started from genomic DNA or PCR products. Since reverse transcriptases are also accepting DNA templates [31], all steps can basically be performed as described within this invention. Optionally also DNA dependent polymerases can be used if DNA is used as template.

As for many analysis methods such as NGS, it is preferable to have defined universal linker sequences present on the 3' and/or 5' end of the cDNA. Such linker sequences can for instance serve as priming sites for PCR amplification to enrich for a library or priming sites for bridge amplification on a solid surface or to prime a sequencing reaction. Therefore it is within the scope of the invention that linker sequences are ligated to the amplified nucleic acid portions. However it is preferred that the 5' linker sequence is directly introduced with the primers (see also Fig. 3 (L1)). Here the 5' linker sequence is a 5' extension to the sequence that is used for priming the polymerase.

Alternatively or in addition a linker sequence can be introduced on the 3' end of the elongated nucleic acid product, e.g. by using a stopper oligo (Fig. 3. (S1, Sm)), that has a linker sequence added on its 3' end (Fig. 3. (L2)). The 5' end of the stopper oligo can be ligated to the 3' end of the extension product (Fig. 3b). The specific ligation reaction ensures that the 5' end of the stopper oligo is not strand displaced. Therefore in a preferred embodiment a stopper oligonucleotide is added to the reverse transcription reaction and wherein the stopper oligonucleotide has a 3' linker sequence extension.

It is preferred that a polymerase is used during elongation that has low or no terminal transferase activity, as to not add non templated nucleotides to the 3' end of the extension product upon reaching the position of the 5' end of the stopper oligo, as a 3' overhang would reduce the specificity and efficiency of the ligation reaction. Reverse transcriptases with low terminal transferase activity are for instance Superscript III (Invitrogen), RTs with no terminal transferease activity are e.g. AMV-RT.

In addition or alternatively an overhang can be digested by a single strand specific nuclease, preferably a 3'-5' exonuclease, such as but not limited to Exonuclease I (3'-5' ssDNA digestion), Exo T5 (3'-5' ss or dsDNA digestion).

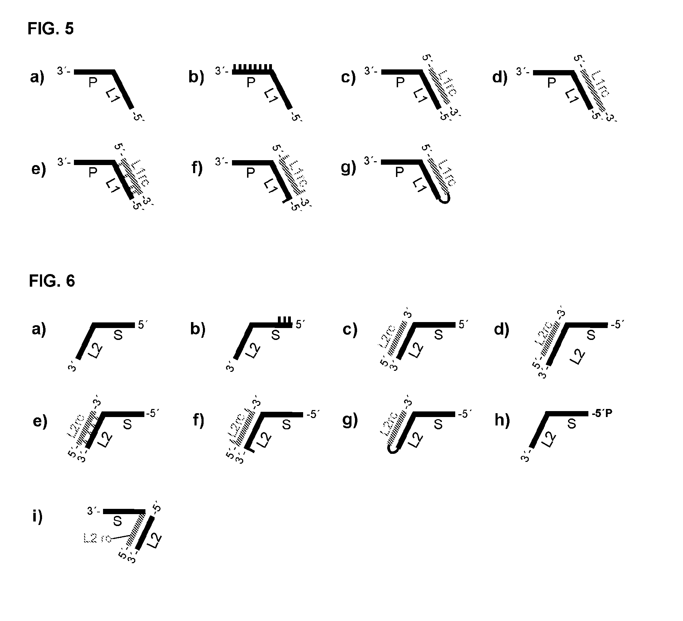

One of the goals of the invention is to control for any bias introduced into the sequence library obtained by the amplified nucleic acid products, and this means in most cases to minimize bias. Linker sequences that are introduced as an extension to random or semi-random priming sequences can also participate in the hybridization to the template. This will add a bias to the library generated. It is therefore preferred that at least the nucleotides of the linker that lay next to the primer or the stopper sequence are inhibited from participating in the hybridization to the template. This can be achieved through different means. For instance an oligonucleotide with a reverse complement sequence to the linker sequence can be added to the reaction (see also Fig. 5c-f; Fig. 6c-f). In that case the reverse complement will compete with the template for hybridization to the linker sequence and by using excess of reverse complement the participation of the linker sequence in priming the RT or hybridizing the stop oligo can be effectively quenched.

It is preferred to provide the reverse complement with nucleotide modifications that enhance the stability of linker:reverse complement hybrid, by using for instance LNAs. However, any other modification can be used that enhances the binding energy (see also Fig. 5e; Fig. 6e).

Furthermore, it is preferred that the primer and/or stopper are added as a premade adapter to the reverse transcription. This means that essentially all linker sequences are in a hybrid with their reverse complement strands and so the linker sequence is inhibited from participation in the hybridization reaction.

Therefore, in a preferred embodiment a reverse complement sequence to the linker sequence part of the oligonucleotides is added to the reaction, preferably already in a hybrid with the oligonucleotide primer and it is further preferred that the Tm of the reverse complement oligo is raised by e.g. introducing modifications such as LNAs, 2' fluoronucleotides or PNAs.

In a most preferred embodiment the reverse complement sequence is covalently linked to the linker sequence. This can be either in direct continuation to the linker sequence or through a nucleotide hairpin or spacers such as C3, C6, C12, or any other moiety. In addition this moiety can be a modification that enhances adapter formation. As described before it is preferred that the nucleotides of the reverse complement are modified to enhance hybridization to the linker sequence. Therefore it is preferred that the reverse complement to the linker sequence is either directly connected to the linker sequence or through a nucleotide hairpin or spacer such as C3, C6, C12 or any other moiety.

As any free 3' OH can potentially serve as an acceptor during polymerization or ligation, it is preferred that any free 3' OH is blocked (see also Fig. 5f; Fig. 6f). Many blocking groups are known in the art. Provided for reference but not limiting are dideoxynucleotides, C-spacers and phosphate groups. In addition, the 3' end of the reverse complement in the priming adapter can also be provided with an overhang (see Fig. 5d). Correspondingly, the linker sequence of the stopping adapter can be provided with a 3' overhang (see Fig. 6d). Therefore it is preferred that the 3'OH of the oligonucleotides that do not participate in the primer extension reaction are blocked and/or where provided in a hybrid that the 3' end has an overhang over the 5' end.

When introducing the linker sequences together with the primer and/or the stopper oligonucleotides the sequence between the linker sequences reflects the template RNA sequence. As a mis-hybridization of the primer or the stopper to the template is much more likely than the incorporation of a wrong nucleotide during polymerization, the primer sequence and/or the stopper sequence are more likely to contain an error than the polymerized sequence. Therefore, when e.g. sequencing the library in an NGS experiment, it would be preferable that the linker sequence that contains the sequencing primer is next to the primer extension product. A solution to this problem is shown in Fig. 4. Here the L2 sequence is in a hybrid with its reverse complement that has been introduced at the 5' side of the stopper oligonucleotide. In this manner the L2 sequence can be ligated to the primer extension product once the polymerase reaches and stops at the stopping oligo. However, the sequence of the stopping sequence of the oligo is not included into the library. Therefore, starting any sequencing reaction from the L2 sequence will not include a potentially mis-hybridized stopper sequence. Therefore, it is preferred that the linker sequence is on the 5' end of the stopping oligonucleotide and the 5' end of a reverse complement to the linker sequence is ligated to the 3' end of a strand displacement stopped extension product as a sequence tag. Thus in preferred embodiments the oligonucleotides primer or oligonucleotide stopper is hybridized with a sequence tag as oligonucleotide label. Especially preferred said sequence tag is preferably hybridized to a portion on the 5' end of said oligonucleotides primer or oligonucleotide stopper. The next, e.g. 1, 2, 3, 4 ,5, 6 or more, nucleotides of said primer or stopper in 3' direction next to the nucleotides of said primer or stopper that are hybridized to said sequence tag are hybridized to the template. This allows positioning of the 5' end of the tag near the 3' end of an elongation product of another primer, that is located upstream of the oligonucleotides primer or oligonucleotide stopper with the hybridized sequence tag so that the elongated 3' end of said further primer can be ligated to the 5' end of said sequence tag. Such tags may also be used for subsequent amplifications reactions of the ligated products and are also referred to as "linkers" herein.
In preferred embodiments the oligonucleotide primer and/or oligonucleotide stopper comprises a nucleotide modification increasing the Tm or stiffening the sugar phosphate backbone of said oligonucleotide. These modifications to increase the Tm are to strengthen hybridization to the template to secure stopping of the elongation reaction and prevent displacement of the primer or stopper. Such modifications are known in the art from oligonucleotide blockers, such as described in GB 2293238, US 2002/0076767 A1, US 6,391,592 B1, WO 99/61661, WO 02/086155, US 5,849,497, WO 2009/019008. Suitable modifications include one or more of the modifications selected from 2'fluoro nucleosides, LNA (locked nucleic acid), ZNA (zip nucleic acids), PNA (Peptide Nucleic Acid). Further the Tm can be increased by using intercalators or additives that specifically bind to nucleic acids, such as Ethidiumbromid, Sybr Green. Preferred intercalators are specific for RNA:DNA hybrids. The number of modified nucleotides may vary, depending on other measures taken to increase the Tm. Preferably 1, 2, 3, 4, 5 or 6 nucleotides are modified. Preferably the modified nucleic acids are on the 5' side of the primer sequence portion, the portion of the oligonucleotide primer or stopper that can hybridize or anneal to the template. Preferably 1, 2 or 3 5' nucleotides are modified. DNA polymerases may have an intrinsic strand displacement activity, especially reverse transcriptases to denature secondary RNA structures. A polymerase having nucleotide strand displacement activity may be used for the elongation.

As DNA polymerases, especially reverse transcriptases, can displace a DNA oligonucleotide from a template strand of RNA at least as good as dissolving secondary or tertiary structure, the hybridization of the oligonucleotide has to be enhanced in order to stop strand displacement of the reverse transcriptase. This can be achieved by using modifications to the oligonucleotide itself or by using additives that either stabilize the hybridization of the oligonucleotide or that stop the reverse transcriptase. Modifications to the oligonucleotides that reduce or inhibit the strand displacement activity of the reverse transcritpase are for instance 2' fluoro nucleosides [13], PNAs [14, see FIG. 1; 15], ZNAs [16, 17], G-Clamps (US 6,335,439, a cytosine analogue capable of Clamp Binding to Guanine) or LNAs (US 2003/0092905; US 7,084,125) [18, 19]. These modifications in general increase the melting temperature of the oligonucleotide, by increasing the local hybridization energy of the oligonucleotide to the template RNA strand. Some also stiffen the sugar phosphate backbone further inhibiting strand displacement by the reverse transcriptase.

Alternatively or in addition, the hybridization of the oligo to the RNA template can be altered by using different additives that bind or intercalate to the nucleic acids. For instance, ethidiumbromide, SybrGreen (US 5,436,134; US 5,658,751; US 6,569,627) or acricidine can be used. Other compounds that can bind to dsNA are actinomycin D and analogues [20]. However they potentially also stabilize RNA secondary structure.

Therefore, it is preferred that such intercalators or additives specifically bind to RNA:DNA hybrids. Examples are aminoglycosides of the Neomycin family (Neomycin, Ribostamycin, Paromomycin and Framycetin [21]). Additives that alter the hybridization properties of the oligonucleotide can also be covalently included into the oligonucleotide structure [21].

The hybridization energy and kinetics can be changed to inhibit the strand displacement by the reverse transcriptase by the addition of nucleic acid binding proteins such as single stranded binding protein such as TtH SSB [22] or Tth RecA [23].

It will be apparent to those skilled in the art that those additives are just examples and any other compound, base modification or enzyme leading to an increased hybridization of the oligonucleotide to RNA can be used to increase the Tm and hence inhibit strand displacement.

The increase in the Tm should be strong enough to prevent a displacement of any one of the 5' end nucleotides of the primer region annealed to the template by an elongating polymerase. In particular, the inventive Tm increase prevents displacement of the 3^{rd}, 2^{nd} and/or 1^{st} nucleotide downstream to the 5' end of the primer region.

In certain embodiments of the invention the strand displacement needs to be stopped right at the first 5' nucleotide of the downstream primer, especially when the cDNA fragments are ligated to each other or to a linker as described below. The strand displacement of an oligonucleotide is reduced or inhibited by using nucleotide modifications that increase the Tm or stiffen the sugar phosphate backbone of oligonucleotide, by e.g. including 2' fluoro nucleosides LNAs, PNAs, ZNAs or PNAs or by using intercallators or additives that specifically bind to nucleic acids such as Ethidiumbromid, Sybr gold, SybrGreen, preferably intercalators that are specific for RNA:DNA hybrids.

Therefore it is preferred that the binding of the oligonucleotide primers are specifically Tm enhanced at their 5' ends to prevent the elongating polymerase from displacing them. Such modifications include but are not limited to LNAs, PNAs, ZNAs, acridine or fluorophores.

Oligonucleotides with an increased Tm at their 5' end such as LNA-modified oligonucleotides enable a stop right at the start of the next primer. It is within the scope of the invention to combine the strand displacement stop by using the LNA-modified oligos together with the displacement synthesis deficient mutants such as Y64A M-MLV or F61W HIV or any other reverse transcriptase with impaired displacement synthesis as well as lowering the reaction temperature and using different additives to increase the binding of oligonucleotides to the RNA.

Preferably C and/or G nucleotides are modified. Even unmodified these nucleotides have a higher Tm than A or T due to increased hydrogen bridge formation when complementary annealed. In preferred embodiments the oligonucleotide primer and/or oligonucleotide stopper comprises at least one, at least 2, at least 3, at least 4, at least 5, at least 6 modified nucleotides being selected from G or C. These modified nucleotides are preferably at the 5' end of the primer sequence as mentioned above.

Most efficient strand displacement stop is achieved by G or C bases as they increase the local Tm of the primer or stopper. Hence semi-random primers or stoppers (hexamers, heptamers, octamers, nonamers, etc.) containing at least two, more preferably three or more Gs or Cs or a combination of Gs and Cs. It is most preferred if these Gs or Cs are modified to increase the local melting temperature, as is the case when using LNA modified bases. It is most preferred that at least 1, at least 2 or at least 3 LNA modified bases are used at the 5' end of the primer. Therefore, it is preferred that at least two, at least 3 modified nucleotides are used optionally chosen from G or C.

Several methods and means exist to ensure that the elongation reaction is stopped when the elongation reaction reaches the position of an oligonucleotide stopper or further or additional primer annealed to the template. This stopping is also referred to as a prevention of strand displacement herein. Strand displacement is a particular problem for reverse transcription due to increased or high strand displacement activities of reverse transcriptases. The inventive step of inhibiting the reverse transcriptase to strand displace the cDNA of an already copied RNA portion ensures that any portion of an RNA molecule that already got copied is not copied again. Therefore, no copied portion of the RNA gets overrepresented in the cDNA library synthesized. This inhibition of strand displacement can be achieved through different means, such as decreasing the reaction temperature, using a reverse transcriptase without strand displacement activity, increasing the melting temperature or the hybridization energy of the primer:RNA hybrid or increasing the rigidity of the RNA or primer or stabilizing the helix. In practice, usually a combination of these means is selected to achieve optimal reaction conditions without strand displacement. A person skilled in the art is well enabled to select suitable parameters as described herein or known in the art to suit a particular template and reaction conditions.

One option is to modify the reaction temperature. In general, a reaction temperature above 37°C is favored during RT for better dissolving secondary structures in the RNA template that leads to a more efficient displacement synthesis. In one embodiment stopping of strand displacement of the primer is achieved by decreasing the reaction temperature. Reaction temperatures below 37°C and down to 4°C are used to reduce strand displacement. It is preferred that the polymerization during RT is carried out between 20°C to 37°C. However, even at these low reaction temperatures the strand displacement stop will not be complete when reverse transcripases are used that have strand displacement activity and /or a simple stopper oligonucleotide is used that has no modifications that alter its melting temperature.

In one embodiment instead of or in addition to decreasing the reaction temperature to achieve a better stop of the elongation at said position of a further primer or a stopper (and reduce strand displacement) reverse transcriptases that are deficient in strand displacement such as the Y64A M-MLV mutant [10] or the F61W (Phe-61-Trp) HIV mutant [11, 12] can be used. Strand displacement deficient mutants are able to displace the next primer or stopper for up to 3nts when unmodified. It is within the scope of the invention to combine the strand displacement stop by decreasing the reaction temperature with the usage of displacement synthesis deficient mutants such as Y64A M-MLV or F61W HIV or any other reverse transcriptase with impaired displacement synthesis.

A drawback of decreasing the reaction temperature during the RT or using a reverse transcriptase that has a reduced or impaired strand displacement activity is that the reverse transcriptase will have difficulties reading through regions of RNA secondary structure. The more stable the secondary structure is the less likely it is that this portion of the RNA gets copied into cDNA. This means that though no part of one RNA molecule gets copied more than once parts of the RNA that form a secondary structure will not be copied. This means that some parts of the RNA will be underrepresented in the cDNA library produced.

Therefore, in a preferred embodiment a reverse transcriptase is used that has strand displacement activity and/or the reverse transcription is carried out at elevated temperatures that sufficiently dissolve secondary RNA structure. Under these conditions every portion of the RNA is accessible to the reverse transcriptase. However as RNA:RNA hybrids are generally more stable than RNA:DNA hybrids, also the cDNA copy can be again strand displaced if no further modifications are used. Therefore, in preferred embodiments the reverse transcription is carried out under conditions that do not allow for secondary or tertiary structure formation of the RNA template (RNA:RNA hybrids) or under conditions that allow for these secondary structures to be strand displaced by the reverse transcriptase, while at the same time the primer extension product (cDNA copy) cannot be displaced.

Increasing the concentration of monovalent counter-ions also will stabilize the hybrid (but also the RNA secondary structure), as it has been reported for HIV-RT that at 75 mM KCl the strand displacement activity is impaired though not inhibited [24, 25]. In a preferred embodiment the concentration of monovalent positive ions is preferably selected from at least 20 mM, at least 30 mM, at least 40 mM, at least 50 mM, at least 60 mM, at least 70 mM. Similar concentrations can be independently selected for single negatively charged ions.

The reverse transcriptase used during the elongation reaction may be a viral reverse transcriptase, and may be selected from the group consisting of AMV RT (and mutants thereof such as Thermoscript RT), MMLV RT (and mutants thereof including but not limited to Superscript I, II or III, Maxima RT, RevertAid, RevertAid Premium, Omniscript, GoScript), HIV RT, RSV RT, EIAV RT, RAV2 RT, Tth DNA polymerase, C. hydrogenoformans DNA polymerase, Avian Sarcoma Leukosis Virus (ASLV) and RNase H - mutants thereof. Mixtures of any of these reverse transcriptases may be used. In particular, mixtures of viral RT enzymes may be used, such as mixtures of MMLV and ASLV, and/or their RNase H reduced or RNase H minus analogs may be used. In any of these methods and compositions, two or more reverse transcriptases may be used, including any reverse transcriptase as described above.

It is within the scope of the invention to combine the strand displacement stop by decreasing the reaction temperature with the usage of displacement synthesis deficient mutants such as Y64A M-MLV or F61W HIV or any other reverse transcriptase with impaired displacement synthesis and increasing the Tm of the oligonucleotide to the RNA.

In any of these methods and compositions a thermostable DNA polymerase may also be used, although with regard to RNA stability and hybridization kinetics of the primers this is not recommended in all situations.

Especially for but not limited to optimal representation in a randomly primed cDNA library the random primers may be present in a concentration from 0.1 µM to 100 µM, and more preferred at about 2.5 µM. In preferred embodiments the ratio (w/w) of primer to template nucleic acids is between 5:1 and 1:1000, preferably between 2:1 and 1:500, preferably between 1:1 and 1:300, preferably between 1:2 and 1:250, preferably between 1:5 and 1:150, preferably between 1:10 and 1:100, preferably between 1:12 and 1:50. The molar ratio of primer to template nucleic acids may be between 1,000:1 to 5,000,000:1, preferably between 5,000:1 to 1,000,000:1, between 10,000:1 to 500,000:1, or between 20,000:1 to 300000:1. In one example, using 150ng of mRNA starting material and assuming an mRNA length of 500-5000nt this would mean primers added at 2.5 µM final concentration are added in a molar excess of 1:280,000-1:28,000. In preferred embodiments the molar or (w/w) ratio of primers to stoppers is between 2:1 and 1:10, preferably between 1:1 and 1:5, especially about 1:2.

When mRNA is reverse transcribed an oligo dT primer can be added to better cover also the poly A tail of the mRNA. Optionally, the addition of the oligo dT primer can be omitted as it is part of the random primer mix, although in a preferred embodiment to guarantee equal representation of the mRNA it should be added. The length of oligo dT can vary from 8 bases to 27 bases, but preferably an 18nt long oligo dT primer is used. Other types of primers with different composition can be used in place of oligo dT. Examples of such compositions include, but are not limited to, oligo dT where the 3' base is A, or C, or G (anchored dT). Furthermore, other sequences or moieties that can base pair with poly A sequences of mRNA can also be used. An example, without limitation, is deoxy uridine, (dU).

The oligo(dT) may consist essentially of between about 12 and about 25 dT residues, and may be an anchored oligo(dT) molecule containing a terminal non-T nucleotide. The oligo(dT) may be oligo(dT) 18-20 or anchored equivalents thereof.

The oligo(dT) may be present in a concentration of between about 20 nM and about 1 µM, most preferably 500 nM are being used. The random primers may be between 5 and 15 nucleotides long, and may be random hexamers with at least 3 LNAs at the 5' site to efficiently stop strand displacement.

An additional feature is that depending on the concentration of the random primer the average length of the product nucleic acids can be influenced. By increasing the concentration of the random primers the resulting elongated product size can be decreased. The desired fragment size depends on the subsequent application. If the desired amplified nucleic acid portions should be below 300nt (as is currently desired for next generation sequencing) the random primer concentrations should range from 2.5 µM to 100 µM final concentration.

According to the methods of the invention the concentration of oligo dT can be 44 nM to 750 nM, for example, or intermediate values. It is evident to those skilled in the art that various ratios of random primers and oligo dT can be used.

When mRNA is under investigation, preferably mRNA enriched RNA samples should be used. Several methods for mRNA enrichment are well documented and known to those skilled in the art. The most commonly used is poly A+ enrichment either by oligodT paramagnetic beads or oligodT cellulose [26]. A number of commercial kits for mRNA enrichment are available. Alternatively mRNA can be enriched by Terminator treatment (Epicentre). Additionally, several companies offer commercially available mRNAs from a number of organisms and tissues.

The concentration and combinations of modified-random primers and oligo dT used in this invention provides efficient and representative conversion of mRNA sequences into cDNA. This method provides superior and non-biased conversion of mRNA sequences into cDNA regardless of the distance from the 3' end of the mRNA. Additionally it guarantees that any RNA molecule is reverse transcribed only once by inhibiting the strand displacement of the reverse transcriptase.

The present invention therefore relates to methods of increasing equal representation of mRNA, and more particularly, to increasing the accuracy of quantification of gene expression. Thus, the present invention provides improved cDNA synthesis useful in gene discovery, genomic research, diagnostics and identification of differentially expressed genes and identification of genes of importance to disease.

In another embodiment oligonucleotide stoppers can be used to specifically block the reverse transcription of unwanted such as but not limited to high abundant or ribosomal transcripts during the reverse transcription process. This step can be used to suppress specific templates to generate a normalized mixture of products. A practical use is in suppressing abundant mRNA to generate a normalized cDNA library. Oligonucleotide stoppers that are used to suppress a template, i.e. are not used to generate a well-defined elongation product are referred herein as blocking oligonucleotides or blockers. Blocking oligonucleotides are known from GB 2293238, US 2002/0076767 A1, US 6,391,592 B1, WO 99/61661, WO 02/086155, US 5,849,497, WO 2009/019008 and can be employed according to the present invention. Preferably such blocking oligonucleotides would be highly specific for those transcripts which should be prevented from being reverse transcribed, hence are preferentially longer than 15nt. Longer oligonucleotides have a higher Tm, hence are harder to be displaced by the reverse transcriptase. In a preferred embodiment the blocking oligonucleotides also have an increased Tm at the 5' end by the introduction of modifications such as but not limited to LNAs, ZNAs, PNAs or acridine. These blocking oligonucleotides will need to be blocked at the 3' end to prevent them from being extended. Such blockages include but are not limited to C3, C6, C12 spacers or dideoxynucleotides. The blocking oligonucleotides should preferentially be designed against a sequence that is located near the 3' end of the RNA, but upstream of the poly A tail.

It is within the scope of the invention to combine the inventive methods by using the above described blocking oligos together with the strand displacement synthesis deficient mutants such as Y64A M-MLV or F61W HIV or any other reverse transcriptase with impaired displacement synthesis as well as lowering the reaction temperature and using different additives to increase the binding of oligonucleotides to the RNA.

Alternatively, if the sequences of the high abundant transcripts are not known, hence specific blocking oligos cannot be designed, the strand displacement stop can also be used for library normalization. A driver library would be synthesized with SDS oligonucleotides (preferentially such with increased local Tm at the 5' end such as LNA, PNA or ZNA modified oligonucleotides) .

With a terminal transferase dideoxynucleotides can be added to those amplified nucleic acid portions in order to generate the blocking oligos. This library can then be hybridized to a tester template sample. High abundant transcripts will have an advantage and the single stranded amplified nucleic acid portion from the driver will be faster and more efficient in hybridizing to the corresponding template. Product synthesis can then be initiated using a primer, such as oligodT primer for mRNA, and since most of the high abundant transcripts are hybridized to blocking oligonucleotides, the low abundant transcripts have a far greater chance of being reverse transcribed. If a template switch or oligo capping protocol is also used, the resulting cDNA of the low abundant transcripts can even be inserted into a PCR reaction with the primers corresponding to the oligodT primer and the template switch or oligo capping nucleotide. Alternatively selective terminal tagging (US 2009/0227009 A1) can be used to tag the 3' end of the newly synthesized cDNA. Since high abundant transcripts are most likely to have never reached full length due to the blocking oligonucleotides only low abundant transcripts will be amplified in a PCR with primers corresponding to the oligodT primer and the 3' cDNA tag.

Alternatively, if the 3' end of the cDNA is not tagged, an oligodT primer with a double stranded T7 RNA polymerase promoter could be used to prime the RT reaction of the normalized cDNA libraries and a linear amplification by in vitro transcription can be used.

Therefore a blocking oligonucleotide can be added to the elongation to stop unwanted sequence portions of a template molecule to be elongated. Pre-selected sequences of the unwanted template sequence are selected for use as blockers.

In a further aspect the present invention relates to the use of the above described methods for generating one or more template nucleic acids to generate a sequence library comprising a mixture of amplified nucleic acid portions of said template nucleic acids. Preferably, the amplified nucleic acid portions provide overlapping sequence portions of the template. This can e.g. be facilitated by using a multitude of different primers that generate various elongated products that in turn are used as amplified nucleic acid portions for the library.

The invention further provides a kit for generating amplified nucleic acid portions of a template nucleic acid as described herein or for generating a sequence library as described herein comprising a DNA polymerase, random oligonucleotide primers which comprise a modification that increases the Tm and random oligonucleotide stoppers that are unsuitable for nucleotide extension and comprise a modification that increases the Tm, optionally further one or more of reaction buffers comprising Mn²⁺ or Mg²⁺, a DNA ligase, a crowding agent suitable for ligase reactions, like PEG. The kit may alternatively comprise DNA polymerase and a DNA ligase and optionally any further compound mentioned above. Kits for use in accordance with the invention may also comprise a carrier means, such as a box or carton, having in close confinement therein one or more container means, such as vials, tubes, bottles and the like. The kit may comprise (in the same or separate containers) one or more of the following: one or more reverse transcriptases, suitable buffers, one or more nucleotides especially dNTPS, and/or one or more primers (e.g., oligo(dT, starters, stoppers, reverse complements, PCR primers) for reverse transcription and subsequent PCR reactions. The kits encompassed by this aspect of the present invention may further comprise additional reagents and compounds necessary for carrying out nucleic acid reverse transcription protocols according to the invention, such as oligodT beads or streptavidin coupled beads. Furthermore, the primers or stoppers may be immobilized on a solid surface.

In preferred embodiments the present invention is defined as follows:
1. Method for generating an amplified nucleic acid portion of a template nucleic acid, comprising
   obtaining said template nucleic acid,
   annealing at least one oligonucleotide primer to said template nucleic acid,
   annealing at least one oligonucleotide stopper to said template nucleic acid,
   elongating the at least one oligonucleotide primer in a template specific manner until the elongating product nucleic acid reaches the position of an annealed oligonucleotide stopper, whereby the elongation reaction is stopped, wherein in said elongation reaction said oligonucleotide stopper is not elongated, and
   wherein the elongated product nucleic acid is ligated to the 5' end of said oligonucleotide stopper.
2. Method of generating an amplified nucleic acid of a template nucleic acid, comprising
   obtaining said template nucleic acid,
   annealing a first oligonucleotide primer to said template nucleic acid,
   annealing at least one further oligonucleotide primer to said template nucleic acid,
   elongating said first oligonucleotide primer in a template specific manner until the elongating product nucleic acid reaches the position of one of said further oligonucleotide primers, whereby the elongation reaction is stopped, and at least one further oligonucleotide primer is elongated in a template specific manner, wherein the stopped elongated product nucleic acid is ligated to the 5' end of said further oligonucleotide primer.
3. The method according to definition 1 or 2, wherein the template nucleic acid is RNA or DNA, preferably RNA.
4. The method according to any one of definitions 1 to 3, further comprising annealing an additional oligonucleotide primer to said template nucleic acid and elongating said additional oligonucleotide primer until the elongating product nucleic acid reaches the position of another oligonucleotide primer or an oligonucleotide stopper, preferably wherein 3, 4, 5, 6, 7, 8, 9, 10, 15, 20, 30, 40, 50, or more different oligonucleotide primers are used, especially preferred wherein the oligonucleotide primers are random primers.
5. The method according to any one of definitions 1 to 4, further comprising annealing an additional oligonucleotide stopper to said template nucleic acid and wherein in said elongation reaction said additional oligonucleotide stopper is not elongated, preferably wherein 2, 3, 4, 5, 6, 7, 8, 9, 10, 15, 20, 30, 40, 50, or more different oligonucleotide stoppers are used, especially preferred wherein the oligonucleotide stoppers are random stoppers.
6. The method according to any one of definitions 1 to 5, characterized in that the any one of the oligonucleotide stoppers comprises a sequence tag, preferably a uniform sequence tag being attached to more than one of the oligonucleotide stoppers, especially preferred to all of the oligonucleotide stoppers, in particular wherein said sequence tag is attached to the 3'end of the oligonucleotide stopper.
7. The method according to any one of definitions 1 to 6, characterized in that any one of the oligonucleotide primers comprises a sequence tag, preferably a uniform sequence tag being attached to more than one of the oligonucleotide primers, especially preferred to all of the oligonucleotide primers, in particular wherein said sequence tag is attached to the 5' end of the oligonucleotide primer.
8. The method according to any one of definitions 1 to 7, characterized in that said labeling step comprises ligation with a sequence tag attached to said oligonucleotide primers or oligonucleotide stoppers or comprises ligation with said oligonucleotide primers or oligonucleotide stoppers, which comprise a sequence tag.
9. The method according to definition 8, wherein said sequence tag is hybridized to the 5' end of said oligonucleotide primer or oligonucleotide stopper, preferably wherein said sequence tag is ligated to the elongation product of an upstream primer.
10. The method according to any one of definitions 1 to 7, characterized in that said labeling step comprises ligation with an oligonucleotide primer or oligonucleotide stopper comprising a sequence tag, preferably wherein said sequence tag is covalently attached to said oligonucleotide primer or oligonucleotide stopper or wherein nucleic acid comprising said sequence tag is hybridized with said oligonucleotide primer or oligonucleotide stopper.
11. The method according to definition 10, wherein said sequence tag is ligated or hybridized to the 3' end of an oligonucleotide stopper.
12. The method according to any one of definitions 8 to 11, wherein a nucleic acid with a reverse complement sequence to the tag sequence is added during the annealing or elongation reaction or is hybridized to the sequence tag.
13. The method according to definition 12, wherein the melting temperature of the sequence tag with the nucleic acid with the reverse complement sequence is increased as compared to unmodified nucleic acid by modified nucleotides, preferably selected from one or more LNA nucleotides, 2' fluoronucleotides or PNA nucleotides.
14. The method according to definition 12 or 13, wherein the nucleic acid with the reverse complement sequence is covalently linked to the sequence tag, preferably via a spacer or hairpin loop.
15. The method according to any one of definitions 8 to 14, further comprising amplifying said elongated products comprising said ligated tag, preferably by PCR using tag specific primers.
16. The method according to any one of definitions 1 to 15, characterized in that the oligonucleotide primers and/or oligonucleotide stoppers are phosphorylated or adenlyated on the 5' end.
17. The method according to any one of definitions 1 to 16, characterized in that the template nucleic acid is immobilized on a solid phase or solid support.
18. The method according to any one of definitions 1 to 17, further comprising the step of washing elongated product nucleic acids, preferably hybridized to a template nucleic acid, especially hybridized to an immobilized template nucleic acid according to definition 17.
19. The method of any one of definitions 1 to 18 characterized in that elongation and ligation reactions are performed concurrently in one reaction step.
20. The method of any one of definitions 1 to 19, wherein said method is performed by addition by a DNA polymerase and/or a DNA ligase, preferably wherein said DNA polymerase and DNA ligase are added in one reaction mixture with the template nucleic acid.
21. The method according to any one of definitions 1 to 20, characterized in that said oligonucleotide primer and/or oligonucleotide stopper comprises a nucleotide modification increasing the Tm or stiffens the sugar phosphate backbone of said oligonucleotide, preferably selected from 2'fluoro nucleosides, LNAs, ZNAs, PNAs, or by using intercalators or additives that specifically bind to nucleic acids, such as Ethidiumbromid, Sybr Green, preferably intercalators that are specific for RNA:DNA hybrids.
22. The method according to definition 21, characterized in that said oligonucleotide primer and/or oligonucleotide stopper comprises at least one, preferably at least 2, more preferred at least 3 modified nucleotides being selected from G or C, preferably at the 5' end of the primer sequence.
23. The method according to any one of definitions 1 to 22, characterized in that a polymerase having nucleotide strand displacement activity is used for the elongation.
24. Use of a method of any one of definitions 1 to 23 to generate a sequence library of one or more template nucleic acids comprising a mixture of, preferably overlapping, amplified nucleic acid portions of said template nucleic acids.
25. Kit for generating amplified nucleic acid portions of a template nucleic acid according to any one of definitions 1 to 23 or for generating a sequence library according to definition 24 comprising a DNA polymerase, random oligonucleotide primers which comprise a modification that increases the Tm and random oligonucleotide stoppers that are unsuitable for nucleotide extension and comprise a modification that increases the Tm, optionally further one or more of reaction buffers comprising Mn²⁺ or Mg²⁺, a DNA ligase, a crowding agent, such as PEG.
26. Kit for generating amplified nucleic acid portions of a template nucleic acid according to any one of definitions 1 to 23 or for generating a sequence library according to definition 24 comprising a DNA polymerase and a DNA ligase, preferably further oligonucleotide primers, oligonucleotide stoppers, a crowding agent, especially PEG, or combinations thereof.

The present invention is further illustrated by the following figures and examples without being limited to these specific embodiments of the invention.

### Figures

### Figure 1: Schematic representation of the principle problem the invention seeks to solve.

a) Primers P1, P2 up to Pn are hybridized to a template RNA. Primer P2 has hybridized to a more upstream (5') position of the template RNA than primer P1 and more generally primer Pn has hybridized to a more upstream position on the template RNA than primer P(n-1). Or in other words, primer P1 has hybridized to a more downstream (3') position on the template than P2 and more generally P(n-1) has hybridized to a more downstream position on the template RNA than Pn. Extension of each primer is initiated by a reverse transcriptase. b) When the reverse transcriptase while polymerizing the extension product of P2 reaches a primer P3, primer P3 and its extension product get strand displaced by the reverse transcriptase that continues to extend the primer P2 extension product. The same is true for the extension product of P1 that displaces primer P2 and its extension product. c) Therefore, when all extension products are finished, one cDNA copy of the template sequence between P1 and P2 is present, but two cDNA copies between P2 and P3 are present. This phenomenon leads to an overrepresentation of the 5' ends of RNA that is primed multiple times, as it happens during standard reverse transcription using random primers such as random hexamers. More generally speaking, when n primers have hybridized and were extended by the polymerase to the 5' end of the template RNA, then the 5' end of the RNA will be represented n times while the 3' end of the RNA will be represented only once.

### Figure 2: Schematic representation of one embodiment of the invention to create a 5' - 3' balanced cDNA library and full length cDNA.

a) The hybridization of primers P1, P2 to Pn is reinforced by using e.g.: locked nucleic acids (LNAs) to inhibit the strand displacement activity of the reverse transcriptase. Here three modifications on the very 5' end of the primers are used. b) Now upon extending a primer that lies more downstream on the template RNA the reverse transcriptase cannot displace the primer that has hybridized to a more upstream position on the template. Therefore, each portion of the RNA is only represented once as cDNA. In that manner no overrepresentation of RNA 5' ends will occur. c) When full length cDNA is desired the individual primer extension products are ligated to yield d) one full length cDNA copy of the template RNA.

### Figure 3: Schematic representation of creating a linker tagged short cDNA library.

For many downstream analyses of cDNA libraries a universal linker sequence is needed either on one or both ends of the cDNA, to for instance amplify the library or start a sequencing reaction. Here the preparation of a library that has two linkers is shown. a) Primers P1, P2 to Pn have a 5' universal linker sequence extension (L1). In addition stopper oligos S1, S2 to Sm are used that have a 3' universal linker sequence extension (L2). The stopper oligos are also modified (e.g. LNAs) so they also cannot be displaced by the reverse transcriptase. b) In a second reaction step the 3' end of the extension product is ligated to the 5' end of the stopper oligo. In that manner a cDNA library is created that has two linker sequences (L1, L2) available in order to c) amplify the whole library during e.g. a subsequent PCR.

### Figure 4: Schematic representation of creating a linker tagged short cDNA library using an alternative stopper oligo concept.

As the error rate that is introduced into the cDNA library through a mis-hybridization of the stopper oligo sequence is greater than if this portion would have been transcribed by a polymerase, an alternative stopper oligo concept is shown. a) Here in comparison to Fig. 3 the stopper oligo Sm is extended on its 5' side by an L2rc (reverse complement) sequence. This L2rc is hybridized with L2 to form an adapter. b) Again as in Fig. 3 the reverse transcriptase is extending the primers Pn until they reach the stopper oligos Sm. During ligation the extension product is now ligated to the L2 strand of the adapter. In this manner again a cDNA library is created that has two linker sequences (L1, L2) present on its ends. Here, however, no stopper oligo sequence is introduced. Therefore, when sequencing from the L2 side of the library no ambiguity towards the identity of the first nucleotides is introduced by a potential mis-hybridization of the stopper oligo.

### Figure 5: Schematic representation of preferred primer modifications.

Depicted are primer modifications that are preferred. These modifications can also be combined. a) General structure of priming oligo, with primer and linker sequence. When generating a randomly primed cDNA library the primer sequence is typically a random sequence such as, for instance, a random hexamer. b) The primer sequence part contains modified nucleotides. The modification reduces or inhibits the strand displacement activity of the reverse transcriptase. c) A reverse complement (L1rc) is introduced that inhibits the L1 sequence part of the oligo to participate in the hybridization to the template RNA strand. Therefore, a bias toward the L1 sequence is blocked. d) To prevent the reverse transcriptase from associating with the linker adaptors and hence lowering the efficiency of reverse transcription a 3' overhang is used at the 3' end of L1rc. e) The L1rc sequence is modified to increase hybridization strength to the L1 sequence to further reduce the likelihood of bias in the library towards the L1 sequence. f) Blocking of all ends that do not participate in polymerase extension or ligase reaction. g) The 5' end of the L1 sequence and the 3' end of the L1rc sequence are connected through a covalent bridge, e.g. a Cn spacer or a hairpin sequence.

### Figure 6: Schematic representation of preferred oligo stopper modifications.

Depicted are stopper oligonucleotide modifications that are preferred. These modifications can also be combined. a) General structure of stopper oligo, with stopper and linker sequence. The stopper sequence is typically a random sequence such as for instance a random nonamer. b) The stopper sequence part contains modified nucleotides. The modification reduces or inhibits the strand displacement activity of the reverse transcriptase. c) A reverse complement (L2rc) is introduced that inhibits the L2 sequence part of the oligo to participate in the hybridization to the template RNA strand. Therefore, a bias toward the L2 sequence is blocked. d) To prevent the reverse transcriptase from associating with the linker adaptors and hence lowering the efficiency of reverse transcription, a 3' overhang is used at the 3' end of L2. e) The L2rc sequence is modified to increase hybridization strength to the L2 sequence to further reduce the likelihood of bias in the library towards the L2 sequence. f) Blocking of all ends that do not participate in polymerase extension or ligase reaction. g) The 5' end of the L1 sequence and the 3' end of the L1rc sequence are connected through a covalent bridge, e.g. a Cn spacer or a hairpin sequence. h) The 5' end of the stopper oligo is phosphorylated to be able to act as a donor in the ligation reaction. Alternatively the 5' end is adenlyated. i) Depicts the general structure of the stopper oligo used in the alternative oligo stopper concept in Fig. 4. The 5' end of the L2 sequence can be phosphorylated to be able to act as a donor in a ligation reaction, or alternatively adenylated.

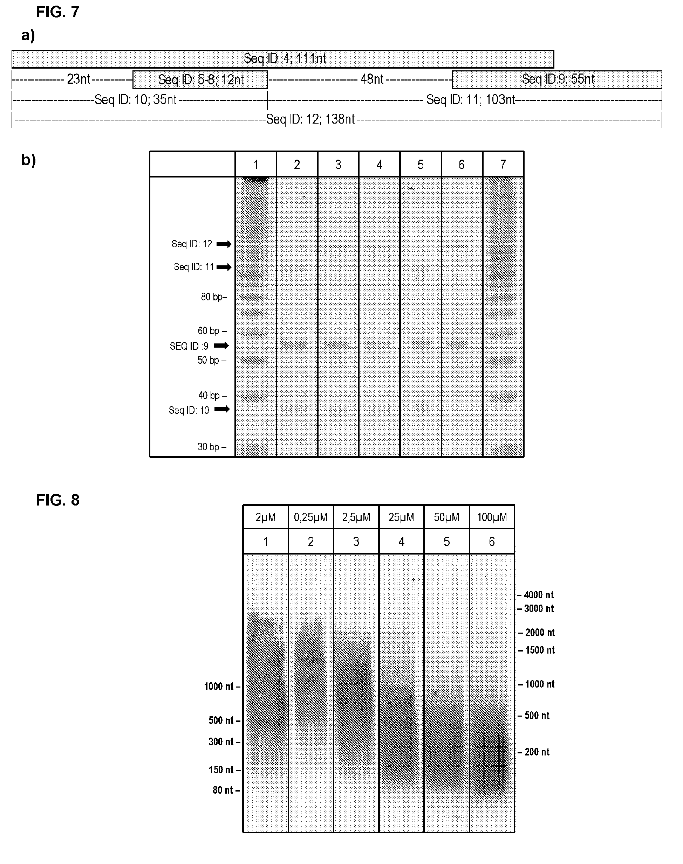

### Figure 7: Stopping of strand displacement during reverse transcription.

a) Illustrates the assay set up for determining the ability of modified or non-modified oligonucleotides (Seq ID No: 5-8) to inhibit the strand displacement activity of the reverse transcriptases which are used in addition to an oligo dT primer (Seq ID No: 9). The extension of the strand displacement stop oligo is 35nt (Seq ID No:10), the strand displacement stop product is 103nt (Seq ID No:11) and the full length cDNA product is 138nt (Seq ID No:12).

b) Shows the results of Example 1.

### Figure 8: Regulation of cDNA fragment size by amount of stop oligos inserted into the RT.

Shows the results of Example 2. cDNA fragments are obtained by random primed cDNA synthesis with or without strand displacement stop oligos and analyzed on an immunoblot.

### Figure 9: Stopping strand displacement during reverse transcription plus ligation of the cDNA fragments to a full length product

Shows the results of Example 3. Here the strand displacement stop is induced by an oligo with 3LNAs at the 5' end and the subsequent ligation of the resulting 2 DNA fragments (35nt, Seq ID No: 10 and 103nt, Seq ID No: 11) to the 138nt full-length product (Seq ID No: 12) using either T4 DNA ligase (lane 3), T4 RNA ligase 2 truncated (lane 5) or a combination of both (lane 4). In lane 6 a control reaction omitting the SDS oligo is shown. In lane 2 the SDS oligo was added, but no ligase was added to the ligation reaction.

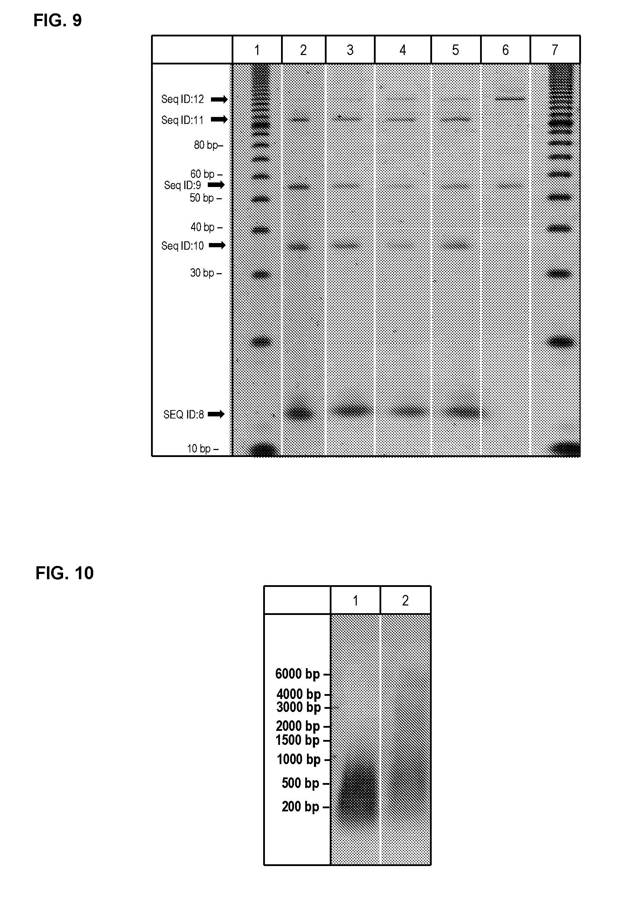

### Figure 10: Validation of SDS/Ligation on mRNA.

Shows the results of Example 4. The immunoblot shows a significant length increase if the short strand displacement stop cDNA fragments are ligated using T4 DNA ligase.

### Figure 11: Strand displacement stop during reverse transcription plus ligation of the cDNA fragments to a full length product results in more product of a selected cDNA.

The results of Example 5 are illustrated. qPCR results of a 5kb fragment from Dync1h1 (NM 030238) amplified from different reverse transcriptions (RTs) are shown. An RT using a SDS oligo (Seq ID No: 15) and an oligo dT primer (Seq ID No: 17) or oligo dT primer (Seq ID No: 17) by itself was performed. T4 DNA ligase was added or in control reactions not added and a qPCR was performed on the resulting cDNAs. Only in case of ligation the SDS oligos were able to produce the 5kb fragment in a PCR reaction (primers Seq ID No:18 and Seq ID No:19). There is more PCR product in reactions performed on SDS/Ligation cDNA (i.e.: more cDNA template available) than in a regular oligo dT primed cDNA (compare lane 2 to lane 6 and 8, respectively).

### Figure 12: cDNA length comparison of SDS/ligation vs oligo dT priming on a 15kb cDNA.

Fig. 12 shows the results of Example 6, a qPCR assay designed to judge the cDNA length generated in an RT reaction. Triangles: oligodT primed reverse transcription, squares: random hexamer primed transcription, circles: inventive transcription with stopped elongation at downstream primers plus ligation.

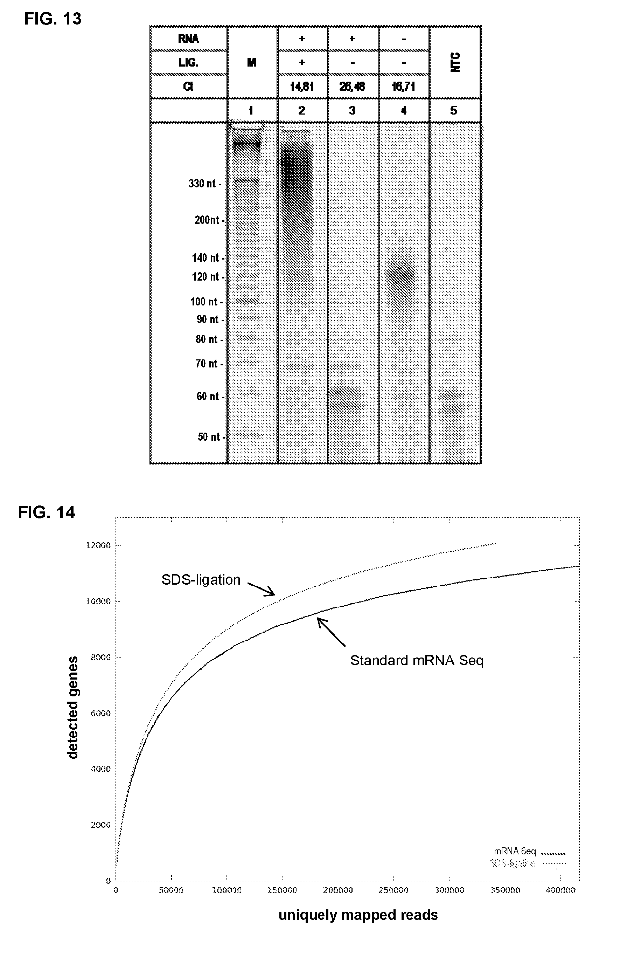

### Figure 13: Generation of a di-tagged DNA libray from mRNA.

Shows a DNA library generated using the SDS/ligation approach (see lane 2), whereas the no ligation control stays empty (see lane 3). Without the addition of RNA template some linker-linker byproducts can be generated since the oligo dT primer will then serve as a template for hybridization (see lane 4). PCR no template controls are clean (see lane 5).

### Figure 14: Discovery blot comparing a library preparation using the new strand displacement stop and ligation protocol (SDS-ligation) with a standard mRNA Seq protocol (TruSeq™ RNA sample prep kit, Catalog # RS-930-20 01). Both were sequenced on an Illumina GAIIx sequencer (single read, 72bp). The X-axis shows the number of reads that uniquely mapped to the annotated genome vs the number of genes discovered on the Y-axis. The comparison of the graphs shows that the SDS-ligation protocol is feasible and actually needs less reads to detect the same amount of genes than the standard protocol.

### Examples

### Abbreviations

qPCR: quantitative polymerase chain reaction

SDS: strand displacement stop

RT: reverse transcription or reverse transcriptase (depending on the context)

LNA: locked nucleic acid

PNA: peptide nucleic acid

rc: reverse complement

Tm: melting temperature

SNP: single nucleotide polymorphism

CGH: comparative genomic hybridization

CNV: copy-number variation

PTO: phosphorothioate bond

Phos: phosphorylation

### Example 1: Strand displacement stop of reverse transcriptases using LNA modified oligonucleotides.

Sequences:
An asterix "*" denotes a phosphorothioate (PTO) bond, a plus "+" in front of a nucleotide denotes a locked nucleic acid (LNA).
"Phos" denotes a phosphorylation,
SEQ ID No. 1: 5'-GCTAATACGACTCACTATAGTTGTCACCAGCATCCC-3'
SEQ ID No. 2: 5'-TTTTTTTTTTTTTTTTTTTTTTTTTTTCGAATGGGCCGCAGGA-3'

SEQ ID No. 3: 5′-
GCTAATACGACTCACTATAGTTGTCACCAGCATCCCTAGACCCGTACAGTGCCCACTCCCCT-

TCCCAGTTTCCGACTGTCCCCGGCCTCCTGCGGCCCATTCGAAAAAAAAAAAAAAAAAAA-
AAAAAA-3′

SEQ ID No. 4: 5'-
guugucaccagcaucccuagacccguacagugcccacuccccuucccaguuccgacugucc-
ccggccuccugcggcccauucgaaaaaaaaaaaaaaaaaaaaaaaaaaa-3' (RNA)

SEQ ID No. 5: (Phos)(5'-+GGGCACTGTACG-3')
SEQ ID No. 6: (Phos)(5'-GGGCACTGTACG-3')
SEQ ID No. 7: (Phos)(5'-G*GGCACTGTAC*G-3')
SEQ ID No. 8: (Phos)(5'-+G+G+GCACTGTAC*G-3') 
SEQ ID No. 9: 5'-
A*CGGAGCCTATCTATATGTTCTTGACATTTTTTTTTTTTTTTTTTTTTTTTTT*T*V-3'
SEQ ID No. 10: 5'-GGGCACTGTACGGGTCTAGGGATGCTGGTGACAAC-3'

SEQ ID No. 11: 5'-
A*CGGAGCCTATCTATATGTTCTTGACATTTTTTTTTTTTTTTTTTTTTTTTTTTTCGAATGGGC
CGCAGGAGGCCGGGGACAGTCGGAAACTGGGAAGGGGAGT-3'

SEQ ID No. 12: 5′-
A*CGGAGCCTATCTATATGTTCTTGACATTTTTTTTTTTTTTTTTTTTTTTTTTTCGAATGGGC
CGCAGGAGGCCGGGGACAGTCGGAAACTGGGAAGGGGAGTGGCACTGTAC-
GGGTCTAGGGATGCTGGTGACAAC-3′

To investigate the feasibility of inhibiting the strand displacement activity of the reverse transcriptase under conditions that enable primer annealing and cDNA polymerization, a proof of concept experiment is shown. For an outline of the assay setup see Fig. 7a, and for results see Fig. 7b.

Generation of in vitro transcribed 111nt RNA template:
A 75bp fragment of GAPDH (NC_000072, 48nt-122nt) was PCR amplified with primers containing either a T7 promoter sequence (Seq ID No: 1) or a T27 tail (Seq ID No: 2). The resulting PCR product (SEQ ID No: 3) served as template for T7 in vitro transcription using Epicentre's AmpliScribe Flash T7 transcription kit. The in vitro transcribed 111nt RNA (Seq ID No: 4) served as template for the strand displacement stop assay during reverse transcription.

cDNA Synthesis:
First-strand cDNA synthesis was carried out using MMLV-H from Promega (250 U/20 µl reaction). The strand displacement assay setup is depicted in Fig. 7a. Primers for cDNA synthesis, LNA-modified oligos (1 LNA-G (Seq ID No: 5), unmodified oligos (Seq ID No: 6), PTO-modified oligos (Seq ID No: 7), 3 LNA-G oligos (Seq ID No: 8) or oligo dT (Seq ID No: 9) were ordered from either Microsynth AG (Balgach Switzerland), or Eurogentec (Seraing, Belgium). 800ng of in vitro transcribed 111nt RNA (Seq ID No: 4) and oligos (Seq ID No: 5-9; 50 nM oligodT primer SEQ ID No:9 and 1.5 µM SEQ ID No:5-8) were heated to 70°C for 2 min with all required components (except for the reverse transcriptase) including: buffer (50 mM Bis-Tris-Methane pH 7.9, 75 mM KC1, 4 mM MgC12, 0.6 M Trehalose and 7.5% glycerol), 0.5 mM each dNTP, 10 mM dithiothreitol (DTT), and slowly annealed by decreasing the temperature to 40°C with 30 sec holds at every 2°C decrease. At 40°C 250 units of reverse transcriptase were added and the temperature was slowly raised to 45°C with 1 min holds at each 1°C increase followed by a 30 minute incubation at 46°C. Following first-strand synthesis, samples were heated to 95°C for 5 min in 0.1 M NaOH, neutralized with equal molarities of HCl and purified by EtOH precipitation. After washing with 75% EtOH the pellets were dissolved in 5µl 10 mM Tris, pH 8.0 and mixed with an equal volume of 100% formamide loading buffer, denatured at 95°C for 2 min, cooled on ice and resolved by electrophoresis in a 15% acrylamide/7M urea.

Results are shown in Fig. 7b.

In the different lanes the second downstream oligo (that has hybridized to a more upstream portion of the template) was varied (Seq ID Nos: 5-8) using an oligo that had one LNA modification (Seq ID No: 5) in lane 2, no modification (Seq ID No: 6) in lane 3, one PTO (Seq ID No: 7) in lane 4 or three LNA modifications (Seq ID No: 8) in lane 5 and no second oligo in lane 6. Lane 1 and 7 show a size marker (10bp marker, Invitrogen). It can be seen that the unmodified (lane 3) or the PTO modified (lane 4) oligonucleotides are completely strand displaced by MMLV-H reverse transcriptase since only the full length product (Seq ID No: 12) at 138nt is visible and no strand displacement stop product at 103nts (Seq ID No: 11). The second oligo has also been extended to 35nts ((Seq ID No: 10) and hence the 5' end of the 111nt RNA is represented twice (once in from of the full length product (138nt) and once by the 35nt extension product of the second oligo). Introducing 1 LNA at the 5' sequence of the 2nd oligo already causes some stop of strand displacement (138nt and 103nt product are visible), while 3 LNAs lead to an almost complete stop of strand displacement (no more 138nt product, just the 103nt strand displacement stop product (Seq ID No: 11).

### Example 2: cDNA fragment size regulation.

This example shows that the size of a cDNA library that is generated from mRNA can be regulated by the concentration of a random primer that stops an elongation reaction. For results see Fig. 8.

RNA Isolation and Purification:
Total RNA from mouse liver was isolated using PeqLab Gold columns in combination with acidic phenol extraction (PeqLab, PEQLAB Biotechnologie GMBH, D-91052 Erlangen) according to manufacturer's recommendation. The amount of RNA was measured by optical absorbance at 260 nm and checked for integrity on a formaldehyde agarose gel or an Agilent Bioanalyzer.

Terminator treatment of total RNA to enrich for mRNAs:
2-5µg of total RNA was treated with Terminator™ 5'-Phosphate-Dependent Exonuclease (Epicentre Biotechnologies, Madison, WI 53713), according to manufacturer's instructions.
cDNA synthesis and immunoblotting:
cDNA synthesis was carried out in 20µl reactions with 50 mM Tris-HCl (pH 8.3 at 25°C), 75 mM KCl, 3 mM MgCl₂, 10 mM DTT, 0.75 mM dNTPs with Digoxigenin-11-2'-deoxy-uridine-5'-triphosphate, alkali-stable. 135 ng of mRNA (terminator treated total RNA) was incubated at 70°C for 2 min in the presence of primer and all the reaction components apart from the enzyme and the dNTPs. A slow annealing program was chosen with 30 seconds holds at the following temperatures: 45°C, 43°C, 40°C, 38°C, 35°C, 30°C, 28°C and 1 minute at 25°C. 200 U of MMLV-H, point mutant (Promega) and dNTPs were added per 20 µl reaction and incubated for 2 min each at the following temperatures: 25°C, 28°C, 30°C, and 35°C before a final extension at 37°C for 10 min. Following first-strand synthesis, samples were heated to 55°C for 15 min in 0.1M NaOH, neutralized with equal molarities of HCl, purified by EtOH precipitation. cDNA fragments were separated by formaldehyde agarose gel electrophoresis (0.8%), transferred to Zeta-Probe GT Genomic Blotting Membranes (BioRAD) by electroblotting for 1 h at 50V according to "Mini Trans-BlotR Electrophoretic Transfer Cell" instruction manual (BioRAD), then crosslinked and by UV-light.

Membranes were equilibrated in 1x blocking buffer (100mM maleic acid/150mM NaCl, pH 7.5) for 5 min, before blocking unspecific binding sites of the membrane in blocking solution (5% milk in blocking buffer) for 30 min. Anti Fab antibodies diluted 1:2,000 (Anti-Digoxigenin-AP FAB fragments, Roche cat# 11 093 274 910); 30 ml blocking solution were incubated for 30 min at room temperature under shaking. Membranes were washed 2x for 15 min in 1x blocking buffer and then equilibrated for 5 min in 1x staining buffer (0.1 M Tris-HCl, pH 9.5 (20°C), 0.1 M NaCl). Staining in staining solution (BCIP^{®}/NBT Liquid Substrate, 800 µl à 30 ml 1x staining buffer) was done at room temperature (in the dark) overnight without shaking.

The results can be seen in Fig. 8.

In lane 1-5 an oligo (SDS-oligo) with three LNAs on its 5' side (SEQ ID No: 13: (Phos)(5'-+N+N+NNNN-3')) was used in increasing concentrations (Lane 2: 0.25 µM; lane 3: 2.5 µM; lane 4: 25 µM, lane 5: 50 µM and lane 6: 100 µM). In Lane 1 a control reaction with a non-modified random hexamer (SEQ ID No: 14:
(Phos)(5'-NNNNNN-3')) at 2 µM is shown. By increasing the amount of SDS oligos the size of the generated cDNA fragments can be decreased.

### Example 3: Ligation using an artificial template.

The example shows that an extension product of a more upstream primer (P1) can be stopped by a more downstream primer (P2) that has three LNA modifications on its 5' end and that the extension product of the upstream primer (P1) can by ligated to the downstream primer (P2) when all are in a hybrid with the template. For an overview of the sequences involved see Fig. 7a. For results see Fig. 9. Reverse transcription was carried out using oligos, template and conditions as described in Example 1.

After the RT the samples were ethanol precipitated and inserted into a 15 µl ligation reaction with 1 mM HCC, 20% PEG-8000, 30 mM Tris-HCl (pH 7.8 at 25°C), 10 mM MgCl₂, 10 mM DTT, 1 mM ATP and 1.5µl of T4 DNA ligase (1-3 Weiss units/µl, Promega) and/or 1µl of T4 RNA ligase 2 truncated (10 units/µl, NEB) and incubated 2 h at 37°C. Unligated small fragments and remaining oligos were removed by PEG precipitation. Therefore the volume of the reaction was increased to obtain 11.5% final PEG concentration and 2µl of linear polyacrylamide (10 mg/ml) were added as carrier. Reactions were thoroughly vortexed before centrifugation at 20,000xg for 15 min at 18°C. The pellet was washed 2x in 75% EtOH, dissolved in 5 µl 10 mM Tris (pH 8.0), mixed with an equal volume of 100% formamide loading buffer, denatured at 95°C for 2 min, cooled on ice and loaded onto a 15% acrylamide/7M urea gel. The results can be seen in Fig. 9. In lane 6 the artificial RNA was reverse transcribed using only an oligo dT primer (SEQ ID No: 9). When an SDS oligo (SEQ ID No: 8) is added in addition to the oligo dT primer (SEQ ID No: 9), there is a complete stop of strand displacement as seen by the appearance of a 103nt SDS product (Seq ID No: 11, see Fig. 9, lane 2). Furthermore a 35nt SDS oligo extension product (Seq ID No: 10) is generated. T4 DNA ligase (Fig. 9, lane 3) or T4 RNA ligase 2, truncated (Fig. 9, lane 5) as well as a combination of both enzymes (Fig. 9, lane 4) were tested for their efficiency to ligate the two cDNA fragments in an RNA hybrid. T4 RNA ligase 2, truncated, is deficient of an adenlyation function and hence can only ligate already adenylated oligos i.e.: oligos that were previously adenylated by T4 DNA ligase in the hybrid or due to a self adenylation process which also happens exclusively in a hybrid. T4 RNA ligase 1 or 2 were not used due to the preferred ligation of single stranded molecules which would then also result in ligation of non-hydridized oligos preferentially to the RNA template. As can be seen in lane 3-5 the stopped products (103nt, Seq ID No: 11) and the SDS oligo extension product (35nt, Seq ID No: 10) can be ligated in the RNA hybrid, resulting in a full-length 138nt cDNA (Seq ID No: 12).

### Example 4: Ligation of cDNA fragments synthesized from poly A-mRNA.

The example shows that by ligating short cDNA fragments that were generated by the inventive method a size shift occurs on the immunoblot, indicating that longer cDNA was created by the ligation process. For methods see Example 2 and 3. For results see Fig. 10. Poly A selected mRNA (mouse liver polyA+mRNA, Stratagene) was used as a template. Short cDNA fragments (see Fig. 10, lane 1, fragments between 100-700 nt) that were generated using combination of oligo dT primer (SEQ ID No: 9) and a random dodecamer with 3 LNA modified nucleotides (SEQ ID No: 15:
(Phos) (5'-+G+G+GHHHNNNNNN-3')) or without LNA modification (SEQ ID No: 16: (Phos) (5'-GGGHHHNNNNNN-3')) were ligated in the RNA hybrid using T4 DNA ligase, which results in long (full-length) cDNAs even longer than 6,000nts (see Fig. 10, lane 2).

### Example 5: In a gene-specific qPCR the SDS/ligation reverse transcription yields more product than a regular oligodT primed RT.

A gene-specific PCR was carried in a 20 µl reaction containing 1 µl cDNA (synthesized from 800 ng total RNA, dissolved in 42 µl 10 mM Tris, pH 8.0 after purification), 50 mM Tris-Cl pH 9.2, 16 mM ammonium sulfate, 0.1% Tween 20, and 5.1 mM MgCl₂, 1.5M Betaine, 1.3% DMSO, 0.5xSYBRGreen I, 0.2 mM of each dNTP, 0.3 µM of each primer (SEQ ID No: 18: 5'-CTGGATGAATGGCTTGAGTGT-3' and SEQ ID No: 19: 5'-GCAACTCCACGCTCATAGAAG-3', primers designed for NM_030238), 0.8 units KlenTaq AC polymerase and 0.2 units Pfu polymerase. Samples were denatured at 95.8°C for 15 sec, and cycled 20 times at 95.8°C for 15 sec, 55°C for 30 sec, 74°C for 20 min (ramp speed at ABI9700: 50%). Subsequently, 19 cycles at 95.8°C for 15 sec, 58°C for 30 sec, 74°C for 20 min (ramp speed at ABI9700: 10%) with a final extension step at 72°C for 3 min followed. PCR products were purified using silica column and were loaded onto a 0.7% agarose gel. Results are shown in Fig. 11. Lane 8 shows the 5096bp PCR product generated from a cDNA synthesized with an oligo dT primer (Seq ID NO: 17: 5'-G*GCGTTTTTTTTTTTTTTTTTT*V-3'). As a control oligodT primed cDNA was also subjected to the ligation protocol that is usually applied for the strand displacement stop oligos (see lane 6). When Seq ID No: 15 (SDS oligos) and Seq ID No: 17 (oligo dT) were used to prime the RT reaction followed by a ligation protocol as described in Example 3, the amount of PCR product generated from such a cDNA was even more than from a regular oligo dT primed cDNA (compare Fig. 11, lane 2 to lane 6 and 8, respectively). This can be explained by the SDS oligos preventing secondary structure formation due to hybridization, whereas those secondary structures lead to premature polymerization stop events if only an oligo dT primer is used. With the SDS/ligation protocol the RT is started at multiple places and the resulting cDNA fragments are then ligated to give full-length cDNA products or in this case the selected 5kb fragment from a randomly chosen specific transcript. Without ligation no PCR product is generated in the subsequent PCR of cDNA synthesized with SDS oligos (see lane 4). This clearly shows that the strand displacement was actually stopped and the generated cDNA fragments were not connected, hence no PCR product can be obtained, since there is no template containing both PCR primer binding sites. Lane 3, 5, and 7 are controls were no reverse transcriptase was added to the RT reaction, hence showing there was no genomic DNA contamination and that the SDS oligos do not result in any unspecific background.

### Example 6: Long transcripts are more efficiently reverse transcribed using a SDS/ligation RT protocol.

A long transcript was chosen (ubiquitin protein ligase E3 component n-recognin 4; Ubr4; NM_001160319) and 200bp amplicons were designed along the cDNA. Amplicons were spaced approximately 2kb apart from each other (primers Seq ID No: 20-29).
SEQ ID NO. 20: 5'-CCTTCCAGGAGGAGTTCATGCCAGT-3'
SEQ ID NO. 21: 5'-CACACGGAGAGATGAATGAGGGGAGA-3'
SEQ ID NO. 22: 5'-GCCTTCATGGCTGTGTGCATTGA-3'
SEQ ID NO. 23: 5'-CATCCTGCCCTGTAGAAAGTCCTCTTG-3'
SEQ ID NO. 24: 5'-CCAGTGTCACAAGTGCAGGTCCATC-3'
SEQ ID NO. 25: 5'-GCGGTCAGCTTTGTCCAGAAGTGTGT-3'
SEQ ID NO. 26: 5'-GTAAGATGGTGGATGGGGTGGGTGT-3'
SEQ ID NO. 27: 5'-TCGCTCTGAAATGCTGACTCCTTCA-3'
SEQ ID NO. 28: 5'-ACCCAGGTTCTACTGCGTCCTGTCC-3'
SEQ ID NO. 29: 5'-CCTCCAGGGCTGTCACGTTCTTCTT-3'

The delta Ct between the more 3' amplicons and the most 5' amplicon (complementary to the 3' end of the mRNA, close to the poly A tail) is used to calculate the fold difference (according to Pfaffl [32]) and hence shows the relative decrease in cDNA generated over the length of the mRNA template. Oligo dT (Seq ID No: 17) primed cDNA was compared to a cDNA primed with Seq ID: 15 and Seq ID No: 14 followed by a ligation of the resulting cDNA fragments in the RNA hybrid. Each of the reaction was performed in triplicates and the means are depicted in Fig. 12. Circles depict values that were measured using SDS-oligos (dashed line); Squares when random hexamers were used (continuous line) and triangles when oligo dT was used (dotted-dashed line). Only the SDS/ligation protocol guarantees a more equal cDNA synthesis over the length of the transcript having an almost perfect 3' to 5' ratio of 1.

Oligo dT primed cDNA (triangles, dotted-dashed line) shows a steady decline in cDNA generated over the length of the 15kb mRNA. Only approximately 10% of the originally started cDNA synthesis reaches 6kb. Random priming without strand displacement leads to an overrepresentation of the 5' end of the mRNA (squares, continuous line).

### Example 7: Generation of a Di-tagged DNA library from mRNA.

0.11 µM Biotin tagged oligo dT primer (SEQ ID NO. 30: (Biotin-TEG)(5'-TTTTTTTTTTTTTTTTTTTTTTTTTTT-3'), 2.5 µM tagged primer(SEQ ID NO. 31: (C12-Spacer) (5'-TCCCTACACGACGCTCTTCCGATCTGACTG+G+G+GNNN-3') + 2.5 µM reverse complement to the primer tag (SEQ ID NO. 32: (C3-Spacer)(5'-CAGTCAGATCG+GAA+GA+GC+GTC+GT+GTAGGGA-3')(C3-Spacer)), 5 µM tagged stopper (SEQ ID NO. 33: (Phos) (5'-+G+G+GHHNNNNAGATCGGAAGAGCGGTTCAGCAGGA-3')(C3Spacer)) + 5 µM reverse complement to the stopper tag (SEQ ID NO. 34: (C12-spacer) (5'-TCCT+GCT+GAACC+GCTCTTCC+GATC-deoxyT-3') , deoxyT denotes a 3' blocked deoxyT), were hybridized to 150 ng of polyA+ mRNA (BioCat Heidelberg, Germany) hybridized in Tris, pH 7.0 (70°C, 1 min, then slowly cooled on ice). Assuming an mRNA length of 500-5,000nt this would mean that the starters are added in a molar excess of 1:280,000-1:28,000, whereas the stoppers are added in a molar excess of 1:560,000-1:56,000. The hybridized nucleic acids were then bound (20 min at room temperature) to pre-washed 1.1 µl Streptavidin coated Dynabeads (M-280, 10 mg/ml). Non-hybridized nucleic acids were washed away (4 washes according to the manufacturer's instructions). Afterwards RT buffer was added to a final concentration of 1x (50 mM Tris-HCl (pH 8.3 at 25°C), 75 mM KCl, 3 mM MgCl₂, 10 mM DTT), 0.5 mM dNTPs, 200 Units MMLV-H as well as 8U of T4 DNA ligase plus 10% PEG and 0.4 µM ATP. The reverse transcription-ligation reaction was performed on beads by heating the reaction slowly raising the temperature (25°C for 2 min, 28°C for 1 min, 31°C for 1 min, 34°C for 1 min) before incubating for 2 hours at 37°C. Again the beads were washed (4x), before hydrolyzing the RNA (55°C for 15 min in 0.1 N NaOH). After neutralization with 0.1 N HCl, samples were precipitated, dissolved in 20µl and 4µl were then inserted into an Illumina qPCR (primers: SEQ ID NO. 35: 5'-A*ATGATACGGCGACCACCGAGATCTACACTCTTTCCCTACACGACGCTCTTCCGATC*T-3' and SEQ ID NO. 36: 5'-C*AAGCAGAAGACGGCATACGAGATCGGTCTCGGCATTCCTGCTGAACCGCTCTTCCGATC*T-3'). Results are shown in Fig. 13. A library smear is generated using the SDS/ligation approach (see lane 2), whereas the no ligation control stays empty (see lane 3). Without the addition of RNA template some linker-linker byproducts can be generated since the oligo dT primer will then serve as a template for hybridization (see lane 4). PCR no template controls are clean (see lane 5).

Example 8: SDS-ligation samples were prepared as described in Example 7. For comparison a standard mRNA-Seq library was prepared according to an Illumina library prep protocol (TruSeq™ RNA sample prep kit, Catalog # RS-930-20 01). Both libraries were sent for NGS sequencing on an Illumina GAIIx machine (single read, 72bp). To compare the performance of both libraries discovery blots were calculated showing the number of detected genes in relation to a given number of reads that mapped uniquely to the annotated genome. The discovery blots are shown in Fig. 14. They show the feasibility of the SDS-ligation protocol that actually outperforms the standard m-RNA Seq library preparation protocol.

### References

[1] Winshell J, Paulson BA, Buelow BD, Champoux JJ. Requirements for DNA unpairing during displacement synthesis by HIV-1 reverse transcriptase. J Biol Chem. 2004 Dec 17;279(51):52924-33. Epub 2004 Sep 30.
[2] Bustin SA, Nolan T. Pitfalls of quantitative real-time reverse-transcription polymerase chain reaction.J Biomol Tech. 2004 Sep;15(3) : 155-66. Review.
[3] Lacey HA, Nolan T, Greenwood SL, Glazier JD, Sibley CP. Gestational profile of Na+/H+ exchanger and Cl-/HCO3- anion exchanger mRNA expression in placenta using real-time QPCR. Placenta. 2005 Jan;26(1) : 93-8.
[4] Zhang J, Byrne CD. Differential priming of RNA templates during cDNA synthesis markedly affects both accuracy and reproducibility of quantitative competitive reverse-transcriptase PCR. Biochem J 1999;337:231-241.
[5] Feinberg, A.P., and Vogelstein, B. A technique for radiolabeling DNA restriction endonuclease fragments to high specific activity. Analytical Biochemistry, 132: 6-13 (1983).
[6] Feinberg, A.P., and Vogelstein, B. A technique for radiolabeling DNA restriction endonuclease fragments to high specific activity. Addendum. Analytical Biochemistry, 137: 266-7 (1984).
[7] Houldsworth, J., and Chaganti, R. Comparative Genomic Hybridization: an Overview. American Journal of Pathology 145: 1253-1260 (1994).
[8] Comparing whole genomes using DNA microarrays. Gresham, D., Dunham, M.J., and Botstein, D. Nature Reviews Genetics 9(4):291-302 (2008).
[9] Metzker ML. Sequencing technologies - the next generation. Nat Rev Genet. 2010 Jan;11(1):31-46. Epub 2009 Dec 8. Review
[10] Paulson BA, Zhang M, Schultz SJ, Champoux JJ. Substitution of alanine for tyrosine-64 in the fingers subdomain of M-MuLV reverse transcriptase impairs strand displacement synthesis and blocks viral replication in vivo. Virology. 2007 Sep 30;366(2):361-76. Epub 2007 May 29.
[11] Fisher TS, Darden T, Prasad VR. Substitutions at Phe61 in the beta3-beta4 hairpin of HIV-1 reverse transcriptase reveal a role for the Fingers subdomain in strand displacement DNA synthesis. J Mol Biol. 2003 Jan 17;325(3):443-59.
[12] Fisher, T. S. & Prasad, V. R. (2002). Substitutions of Phe61 located in the vicinity of template 50-overhang influence polymerase fidelity and nucleoside analog sensitivity of HIV-1 reverse transcriptase. J. Biol. Chem. 277, 22345-22352.
[13] Kawasaki, A.M., et al., Uniformly modified 2'-deoxy-2'-fluoro phosphorothioate oligonucleotides as nuclease resistant antisense compounds with high affinity and specificity for RNA targets, Journal of Medicinal Chemistry (1993), 36: 831-841.
[14] Nielsen PE, Egholm M, Berg RH, Buchardt O. Sequence-selective recognition of DNA by strand displacement with a thymine-substituted polyamide. Science. 1991 Dec 6;254(5037):1497-500.
[15] Egholm M, Buchardt O, Christensen L, Behrens C, Freier SM, Driver DA, Berg RH, Kim SK, Norden B, Nielsen PE. PNA hybridizes to complementary oligonucleotides obeying the Watson-Crick hydrogen-bonding rules. Nature. 1993 Oct 7;365(6446):566-8.
[16] Voirin, E. et al. (2007) Versatile synthesis of oligodeoxyribonucleotide-oligospermine conjugates. Nat Protoc, 2, 1360-1367
[17] Moreau et al. (2009) Zip nucleic acids (ZNAs): new high affinity oligonucleotides as potent primers for PCR and reverse transcription. Nucl. Acids Res., 37: e130*; doi:10.1093*/*nar*/*gkp661.*
[18] Nielsen, P., Pfundheller, H. M., Olsen, C. E. and Wengel, J., Synthesis of 2'-0,3'-C-Linked Bicyclic Nucleosides and Bicyclic Oligonucleotides, J. Chem. Soc., Perkin Trans. 1, 1997, 3423;
[19] Singh, S. K.; Nielsen, P.; Koshkin, A. A.; Wengel, J. LNA (Locked Nucleic Acids): Synthesis and High-Affinity Nucleic Acid Recognition. Chem. Commun. 1998, 455-456.
[20] Takusagawa, F. (1997) Selectivity of F8-actinomycin D for RNA:DNA hybrids and its anti-leukemia activity. Bioorg. Med. Chem. 5, 1197-1207.
[21] Nicholas N. Shaw, Dev P. Arya* Recognition of the unique structure of DNA:RNA hybrid; review Biochimie 90 (2008), 1026-1039
[22] Perales C, Cava F, Meijer WJ, Berenguer J. Enhancement of DNA, cDNA synthesis and fidelity at high temperatures by a dimeric single-stranded DNA-binding protein. Nucleic Acids Res. 2003 Nov 15;31(22):6473-80.
[23] Shigemori Y, Mikawa T, Shibata T, Oishi M. Multiplex PCR: use of heat-stable Thermus thermophilus RecA protein to minimize non-specific PCR products. Nucleic Acids Res. 2005 Aug 8;33(14):e126.
[24] Boyer PL, Sarafianos SG, Arnold E, Hughes SH. Analysis of mutations at positions 115 and 116 in the dNTP binding site of HIV-1 reverse transcriptase. Proc Natl Acad Sci USA. 2000 Mar 28;97(7):3056-61.
[25] Fuentes GM, Rodriguez-Rodriguez L, Palaniappan C, Fay PJ, Bambara RA. Strand displacement synthesis of the long terminal repeats by HIV reverse transcriptase. J Biol Chem. 1996 Jan 26;271(4):1966-71.
[26] Sambrook J.& Russell D., Molecular Cloning: A Laboratory Manual (Third Edition, book 1, chapter 7.20) US6335439: Method of preparing phosphoramidites
[27] Engler, M. J. and Richardson, C. C. (1982) DNA ligases. In The Enzymes, vol. XV (Boyer, P. D., ed.), pp. 3-29, Academic Press, New York
[28] Hsuih TC, Park YN, Zaretsky C, Wu F, Tyagi S, Kramer FR, Sperling R, Zhang DY. Novel, ligation-dependent PCR assay for detection of hepatitis C in serum. J Clin Microbiol. 1996 Mar;34(3):501-7.
[29] Bullard DR, Bowater RP. Direct comparison of nick-joining activity of the nucleic acid ligases from bacteriophage T4. Biochem J. 2006 Aug 15;398(1):135-44.
[30] Zimmerman SB, Pheiffer BH. Macromolecular crowding allows blunt-end ligation by DNA ligases from rat liver or Escherichia coli. Proc Natl Acad Sci USA. 1983 Oct;80(19):5852-6.
[31] Gerard G. F., and D' Alessio J. M., Chapter 6 (73-93) From: Methods in Molecular Biology, Vol.16: Enzymes of Molecular Biology Edited by: M. M. Burell 1993 Humana Press Inc. Totowa, NJ
[32] Pfaffl MW. A new mathematical model for relative quantification in real-time RT-PCR. Nucleic Acids Res. 2001 May 1;29(9):e45.
   US6335439. Alessandra Eleuteri et al. (2002): Method of preparing phosphoramidites
   US20030092905. Alexei Kochkine (2003): Synthesis of [2.2.1]bicyclo nucleosides
   US7084125. Jesper Wengel (2006): Xylo-LNA analogues US5436134.Richard P. Haugland et al. (1995): Cyclic-substituted unsymmetrical cyanine dyes.
   US5658751 Stephen T. Yue et al. (1997): Substituted unsymmetrical cyanine dyes with selected permeability. Dye No. 211. US6569627. Carl T. Wittwer (2003): Monitoring hybridization during PCR using SYBR™ Green I
   US2009/0227009 A1. Roy R. Sooknanan (2009): SELECTIVE TERMINAL TAGGING OF NUCLEIC ACIDS

## Claims

1. Method for generating an amplified nucleic acid portion of a template nucleic acid, comprising
obtaining said template nucleic acid,
annealing at least one oligonucleotide primer to said template nucleic acid,
annealing at least one oligonucleotide stopper to said template nucleic acid,
elongating the at least one oligonucleotide primer in a template specific manner until the elongating product nucleic acid reaches the position of an annealed oligonucleotide stopper, whereby the elongation reaction is stopped, wherein in said elongation reaction said oligonucleotide stopper is not elongated, and
wherein the elongated product nucleic acid is ligated to the 5' end of said oligonucleotide stopper.

2. Method of generating an amplified nucleic acid of a template nucleic acid, comprising
obtaining said template nucleic acid,
annealing a first oligonucleotide primer to said template nucleic acid,
annealing at least one further oligonucleotide primer to said template nucleic acid,
elongating said first oligonucleotide primer in a template specific manner until the elongating product nucleic acid reaches the position of one of said further oligonucleotide primers, whereby the elongation reaction is stopped, and at least one further oligonucleotide primer is elongated in a template specific manner, wherein the stopped elongated product nucleic acid is ligated to the 5' end of said further oligonucleotide primer.

3. The method according to claim 1 or 2, wherein the template nucleic acid is RNA or DNA, preferably RNA.

4. The method according to any one of claims 1 to 3, further comprising annealing an additional oligonucleotide primer to said template nucleic acid and elongating said additional oligonucleotide primer until the elongating product nucleic acid reaches the position of another oligonucleotide primer or an oligonucleotide stopper, preferably wherein 3, 4, 5, 6, 7, 8, 9, 10, 15, 20, 30, 40, 50, or more different oligonucleotide primers are used, especially preferred wherein the oligonucleotide primers are random primers.

5. The method according to any one of claims 1 to 4, further comprising annealing an additional oligonucleotide stopper to said template nucleic acid and wherein in said elongation reaction said additional oligonucleotide stopper is not elongated, preferably wherein 2, 3, 4, 5, 6, 7, 8, 9, 10, 15, 20, 30, 40, 50, or more different oligonucleotide stoppers are used, especially preferred wherein the oligonucleotide stoppers are random stoppers.

6. The method according to any one of claims 1 to 5, **characterized in that** any one of the oligonucleotide stoppers comprises a sequence tag, preferably a uniform sequence tag being attached to more than one of the oligonucleotide stoppers, especially preferred to all of the oligonucleotide stoppers, in particular wherein said sequence tag is attached to the 3'end of the oligonucleotide stopper.

7. The method according to any one of claims 1 to 6, **characterized in that** the any one of the oligonucleotide primers comprises a sequence tag, preferably a uniform sequence tag being attached to more than one of the oligonucleotide primers, especially preferred to all of the oligonucleotide primers, in particular wherein said sequence tag is attached to the 5' end of the oligonucleotide primer.

8. The method according to any one of claims 1 to 7, **characterized in that** said labeling step comprises ligation with a sequence tag attached to said oligonucleotide primers or oligonucleotide stoppers or comprises ligation with said oligonucleotide primers or oligonucleotide stoppers, which comprise a sequence tag.

9. The method according to claim 8, further comprising amplifying said elongated products comprising said ligated tag, preferably by PCR using tag specific primers.

10. The method according to any one of claims 1 to 9, **characterized in that** the oligonucleotide primers and/or oligonucleotide stoppers are phosphorylated or adenlyated on the 5' end.

11. The method according to any one of claims 1 to 10, **characterized in that** said oligonucleotide primer and/or oligonucleotide stopper comprises a nucleotide modification increasing the Tm or stiffening the sugar phosphate backbone of said oligonucleotide, preferably selected from 2'fluoro nucleosides, LNAs, ZNAs, PNAs, or by using intercalators or additives that specifically bind to nucleic acids, such as Ethidiumbromid, Sybr Green, preferably intercalators that are specific for RNA:DNA hybrids.

12. The method according to claim 11, **characterized in that** said oligonucleotide primer and/or oligonucleotide stopper comprises at least one, preferably at least 2, more preferred at least 3 modified nucleotides being selected from G or C, preferably at the 5' end of the primer sequence.

13. The method according to any one of claims 1 to 12, **characterized in that** a polymerase having nucleotide strand displacement activity is used for the elongation.

14. Use of a method of any one of claims 1 to 13 to generate a sequence library of one or more template nucleic acids comprising a mixture of, preferably overlapping, amplified nucleic acid portions of said template nucleic acids.

15. Kit for generating amplified nucleic acid portions of a template nucleic acid according to any one of claims 1 to 13 or for generating a sequence library according to claim 14 comprising a DNA polymerase, random oligonucleotide primers which comprise a modification that increases the Tm and random oligonucleotide stoppers that are unsuitable for nucleotide extension and comprise a modification that increases the Tm, optionally further one or more of reaction buffers comprising Mn²⁺ or Mg²⁺, a DNA ligase, a crowding agent.


SEQUENCE LISTING

<110>  Lexogen GmbH

<120>  Nucleic acid transcription method

<130>  r59519

<160>  36

<170>  PatentIn version 3.3

<210>  1
<211>  36
<212>  DNA
<213>  Artificial

<220>
<223>  primer oligonucleotide

<400>  1
gctaatacga ctcactatag ttgtcaccag catccc                                36

<210>  2
<211>  43
<212>  DNA
<213>  Artificial

<220>
<223>  primer oligonucleotide

<400>  2
tttttttttt tttttttttt tttttttcga atgggccgca gga                        43

<210>  3
<211>  130
<212>  DNA
<213>  Artificial

<220>
<223>  PCR product

<400>  3
gctaatacga ctcactatag ttgtcaccag catccctaga cccgtacagt gcccactccc      60

cttcccagtt tccgactgtc cccggcctcc tgcggcccat tcgaaaaaaa aaaaaaaaaa     120

aaaaaaaaaa                                                            130

<210>  4
<211>  111
<212>  RNA
<213>  Artificial

<220>
<223>  PCR product

<400>  4
guugucacca gcaucccuag acccguacag ugcccacucc ccuucccagu uuccgacugu      60

ccccggccuc cugcggccca uucgaaaaaa aaaaaaaaaa aaaaaaaaaa a              111


<210>  5
<211>  12
<212>  DNA
<213>  Artificial

<220>
<223>  stopper oligonucleotide


<220>
<221>  LNA
<222>  (1)..(1)

<400>  5
gggcactgta cg                                                          12


<210>  6
<211>  12
<212>  DNA
<213>  Artificial

<220>
<223>  stopper oligonucleotide

<400>  6
gggcactgta cg                                                          12


<210>  7
<211>  11
<212>  DNA
<213>  Artificial

<220>
<223>  stopper oligonucleotide


<220>
<221>  phosphorothioate backbone
<222>  (1)..(2)

<220>
<221>  phosphorothioate backbone
<222>  (10)..(11)

<400>  7
gggcactgta c                                                           11


<210>  8
<211>  11
<212>  DNA
<213>  Artificial

<220>
<223>  stopper oligonucleotide

<220>
<221>  LNA
<222>  (1)..(3)

<220>
<221>  phosphorothioate backbone
<222>  (10)..(11)

<400>  8
gggcactgta c                                                            11

<210>  9
<211>  55
<212>  DNA
<213>  Artificial

<220>
<223>  primer oligonucleotide

<400>  9
acggagccta tctatatgtt cttgacattt tttttttttt tttttttttt ttttv            55

<210>  10
<211>  35
<212>  DNA
<213>  Artificial

<220>
<223>  PCR product

<400>  10
gggcactgta cgggtctagg gatgctggtg acaac                                  35

<210>  11
<211>  103
<212>  DNA
<213>  Artificial

<220>
<223>  PCR product

<400>  11
acggagccta tctatatgtt cttgacattt tttttttttt tttttttttt ttttcgaatg      60

ggccgcagga ggccggggac agtcggaaac tgggaagggg agt                        103

<210>  12
<211>  138
<212>  DNA
<213>  Artificial

<220>
<223>  PCR product

<400>  12
acggagccta tctatatgtt cttgacattt tttttttttt tttttttttt ttttcgaatg      60

ggccgcagga ggccggggac agtcggaaac tgggaagggg agtgggcact gtacgggtct     120

agggatgctg gtgacaac                                                   138


<210>  13
<211>  6
<212>  DNA
<213>  Artificial

<220>
<223>  random hexamer oligonucleotide


<220>
<221>  LNA
<222>  (1)..(3)

<220>
<221>  misc_feature
<222>  (1)..(6)
<223>  n is a, c, g, or t

<400>  13
nnnnnn                                                                  6


<210>  14
<211>  6
<212>  DNA
<213>  Artificial

<220>
<223>  random hexamer oligonucleotide


<220>
<221>  misc_feature
<222>  (1)..(6)
<223>  n is a, c, g, or t

<400>  14
nnnnnn                                                                  6


<210>  15
<211>  12
<212>  DNA
<213>  Artificial

<220>
<223>  primer oligonucleotide

<220>
<221>  LNA
<222>  (1)..(3)

<220>
<221>  misc_feature
<222>  (7)..(12)
<223>  n is a, c, g, or t

<400>  15
ggghhhnnnn nn                                                          12


<210>  16
<211>  12
<212>  DNA
<213>  Artificial

<220>
<223>  primer oligonucleotide


<220>
<221>  misc_feature
<222>  (7)..(12)
<223>  n is a, c, g, or t

<400>  16
ggghhhnnnn nn                                                          12


<210>  17
<211>  23
<212>  DNA
<213>  Artificial

<220>
<223>  primer oligonucleotide

<400>  17
ggcgtttttt tttttttttt ttv                                              23


<210>  18
<211>  21
<212>  DNA
<213>  Artificial

<220>
<223>  primer oligonucleotide

<400>  18
ctggatgaat ggcttgagtg t                                                21


<210>  19
<211>  21
<212>  DNA
<213>  Artificial

<220>
<223>  primer oligonucleotide

<400>  19
gcaactccac gctcatagaa g                                                21


<210>  20
<211>  25
<212>  DNA
<213>  Artificial

<220>
<223>  primer oligonucleotide

<400>  20
ccttccagga ggagttcatg ccagt                                            25


<210>  21
<211>  26
<212>  DNA
<213>  Artificial

<220>
<223>  primer oligonucleotide

<400>  21
   cacacggaga gatgaatgag gggaga                                            26


<210>  22
<211>  23
<212>  DNA
<213>  Artificial

<220>
<223>  primer oligonucleotide

<400>  22
gccttcatgg ctgtgtgcat tga                                              23


<210>  23
<211>  27
<212>  DNA
<213>  Artificial

<220>
<223>  primer oligonucleotide

<400>  23
catcctgccc tgtagaaagt cctcttg                                          27


<210>  24
<211>  25
<212>  DNA
<213>  Artificial

<220>
<223>  primer oligonucleotide

<400>  24
ccagtgtcac aagtgcaggt ccatc                                          25


<210>  25
<211>  26
<212>  DNA
<213>  Artificial

<220>
<223>  primer oligonucleotide

<400>  25
gcggtcagct ttgtccagaa gtgtgt                                         26


<210>  26
<211>  25
<212>  DNA
<213>  Artificial

<220>
<223>  primer oligonucleotide

<400>  26
gtaagatggt ggatggggtg ggtgt                                          25


<210>  27
<211>  25
<212>  DNA
<213>  Artificial

<220>
<223>  primer oligonucleotide

<400>  27
tcgctctgaa atgctgactc cttca                                          25


<210>  28
<211>  25
<212>  DNA
<213>  Artificial

<220>
<223>  primer oligonucleotide

<400>  28
acccaggttc tactgcgtcc tgtcc                                          25


<210>  29
<211>  25
<212>  DNA
<213>  Artificial

<220>
<223>  primer oligonucleotide

<400>  29
cctccagggc tgtcacgttc ttctt                                             25


<210>  30
<211>  27
<212>  DNA
<213>  Artificial

<220>
<223>  primer oligonucleotide

<400>  30
   tttttttttt tttttttttt ttttttt                                             27


<210>  31
<211>  36
<212>  DNA
<213>  Artificial

<220>
<223>  primer oligonucleotide


<220>
<221>  misc_feature
<222>  (34)..(36)
<223>  n is a, c, g, or t

<400>  31
tccctacacg acgctcttcc gatctgactg gggnnn                                 36


<210>  32
<211>  30
<212>  DNA
<213>  Artificial

<220>
<223>  primer oligonucleotide

<400>  32
cagtcagatc ggaagagcgt cgtgtaggga                                        30


<210>  33
<211>  34
<212>  DNA
<213>  Artificial

<220>
<223>  stopper oligonucleotide


<220>
<221>  misc_feature

<222>  (6)..(9)
<223>  n is a, c, g, or t

<400>  33
ggghhnnnna gatcggaaga gcggttcagc agga                                34


<210>  34
<211>  25
<212>  DNA
<213>  Artificial

<220>
<223>  stopper oligonucleotide

<400>  34
tcctgctgaa ccgctcttcc gatct                                          25


<210>  35
<211>  58
<212>  DNA
<213>  Artificial

<220>
<223>  primer oligonucleotide

<400>  35
aatgatacgg cgaccaccga gatctacact ctttccctac acgacgctct tccgatct      58


<210>  36
<211>  61
<212>  DNA
<213>  Artificial

<220>
<223>  primer oligonucleotide

<400>  36
caagcagaag acggcatacg agatcggtct cggcattcct gctgaaccgc tcttccgatc    60

t                                                                    61